# EUROPEAN PATENT SPECIFICATION

(11) **EP 1 658 282 B1**
(45) Date of publication and mention of the grant of the patent: **27.10.2010**
(21) Application number: 04767016.1
(22) Date of filing: 27.08.2004
(51) Int. Cl.: C07D 405/04, C07D 405/14, C07D 471/16, C07F 9/48, G01N 33/52, G01N 33/58

(54) **NOVEL CHELATING AGENTS AND CHELATES AND THEIR USE**
NEUE CHELATBILDNER UND CHELATE UND DEREN VERWENDUNG
NOUVEAUX AGENTS CHELATEURS, NOUVEAUX CHELATES ET LEUR UTILISATION

(30) Priority: 29.08.2003 US 498704 P; 29.08.2003 FI 20031221
(43) Date of publication of application: 24.05.2006
(73) Proprietor: WALLAC OY, 20750 Turku (FI)
(72) Inventor: HOVINEN, Jari, FIN-21200 Raisio (FI); MUKKALA, Veli-Matti, FIN-20780 Kaarina (FI); HAKALA, Harri, FIN-20740 Turku (FI); PEURALAHTI, Jari, 20610 Turku (FI)
(74) Representative: Nylund, Solveig Helena
(86) International application number: PCT/FI2004/000502
(87) International publication number: WO 2005/021538

(56) References cited:
- EP-A1- 0 967 205
- WO-A1-89/08263
- WO-A1-97/40055
- US-A- 4 925 804
- MUKKALA VELI-MATTI ET AL.: '119. New 2,2'-bipyridine derivatives and their luminescence properties with europium(III) and trebium(III) ions1)' HELVETICA CHIMICA ACTA vol. 75, 1992, pages 1578 - 1592, XP002917922
- MUKKALA V.-M. ET AL.: 'New fluorescent Eu(III) and Tb(III) chelates of 2,2'-bipyridine derivatives' EUR. J. SOLID STATE INORG. CHEM. vol. 29, 1992, pages 53 - 56, XP002983298
- LATVA MARTTI ET AL.: 'Correlation between the lowest triplet state energy level of the ligand and lanthanide(III) luminescence quantum yield' JOURNAL OF LUMINESCENCE vol. 75, 1997, pages 149 - 169, XP001012267
- MUKKALA VELI-MATTI ET AL.: '85. Development of luminescent europium(III) and terbium(III) chelates of 2,2':6',2''-terpyridine derivatives for protein labelling' HELVETICA CHIMICA ACTA vol. 76, 1993, pages 1361 - 1378, XP002082606

## Description

### FIELD OF THE INVENTION

This invention relates to a group of novel chelating agents, novel chelates, biomolecules labeled with said chelates or chelating agents as well as solid supports conjugated with said chelates, chelating agents or labeled biomolecules.

### BACKGROUND OF THE INVENTION

The publications and other materials used herein to illuminate the background of the invention, and in particular, cases to provide additional details respecting the practice, are incorporated by reference.

Because of their unique luminescence properties lanthanide(111) chelates are often used as non-radioactive markers in a wide variety of routine and research applications. Since lanthanide(III) chelates give strong, long decay-time luminescence, they are ideal labels for assays where high sensitivity is required. Time-resolved fluorometric assays based on lanthanide chelates have found increasing applications in diagnostics, research and high throughput screening. The heterogeneous DELFIA^{®} technique is applied in assays requiring exceptional sensitivity, robustness and multi-label approach [Hemmilä et al. Anal. Biochem. 1984, 137, 335-343]. Development of highly luminescent stable chelates extends the use of time resolution to homogeneous assays, based on fluorescence resonance energy transfer (TR-FRET), fluorescence quenching (TR-FQA) or changes in luminescence properties of a chelate during a binding reaction [Hemmilä, I.; Mukkala, V.-M. Crit. Rev. Clin. Lab. Sci. 2001, 38, 441-519].

Most commonly the conjugation reaction is performed in solution between an amino or mercapto group of a bioactive molecule (such as protein, peptide, nucleic acid, oligonucleotide or hapten) and isothiocyanato, haloacetyl, 3,5-dichloro-2,4,6-triazinyl derivatives of lanthanide(III) chelates, as well as other reporter groups. Since in all the cases the labeling reaction is performed with an excess of an activated label, laborious purification procedures cannot be avoided. Especially, when attachment of several label molecules, or site-specific labeling in the presence of several functional groups of similar reactivities is required, the isolation and characterization of the desired biomolecule conjugate is extremely difficult, and often practically impossible. Naturally, solution phase labeling of large biomolecules, such as proteins cannot be avoided. In these cases, the labeling reaction has to be as selective and effective as possible.

A number of attempts have been made to develop new highly luminescent chelate labels suitable for time-resolved fluorometric applications. These include e.g. stabile chelates composed of derivatives of pyridines [US 4,920,195, US 4,801,722, US 4,761,481, PCT/FI91/00373, US 4,459,186, EP A-077060, Remuinan et al, J. Chem. Soc. Perkin Trans 2, 1993, 1099], bipyridines [US 5,216,134], terpyridines [US 4,859,777, US 5,202,423, US 5,324,825] or various phenolic compounds [US 4,670,572, US 4,794,191, Ital Pat. 42508 A789] as the energy mediating groups and polycarboxylic acids as chelating parts. In addition, various dicarboxylate derivatives [US 5,032,677, US 5,055,578, US 4,772,563] macrocyclic cryptates [US 4,927,923, WO 93/5049, EP-A-493745] and macrocyclic Schiff bases [EP-A-369-000] have been disclosed. Also a method for the labelling ofbiospecific binding reactant such as hapten, a peptide, a receptor ligand, a drug or PNA oligomer with luminescent labels by using solid-phase synthesis has been published [US 6,080,839]. Similar strategy has also been exploited in multilabeling of oligonucleotides on solid phase [EP A 1152010, EP A 1308452].

Publication WO8908263 discloses the synthesis and photochemical properties of some lanthanide(III) chelates, including chelates comprising furylpyridine subunits. The chelates according to publication WO890826 have the furylpyridine subunits linked directly to each other or they are linked together with a bridge allowing electron delocalization between the pyridine rings and simultaneously coordination of the ring nitrogens with a chelated metal ion so as to form a five or a six membered ring.

However, no chelates or chelating agents having furylsubstituted pyridyl groups in their chromophoric moiety and having a reactive group enabling binding to a biomolecule or to a solid phase have been described before.

### OBJECTS AND SUMMARY OF THE INVENTION

The main object of the present invention is to provide chelating agents and metal chelates thereof, useful for labeling biomolecules for use as probes in time resolved fluorescence spectroscopy, .

A particular object of this invention is to provide a chelating agent which gives a very strong fluorescense with different chelated lanthanide ions, particularly with europium (III), samarium (III), terbium (III) and dysprosium (III). Such lanthanide chelates are especially useful in multiparameter bioaffinity assays and in high-throughput screening of drug candidates.

A further object of this invention is to provide chelating agents giving rise to metal chelates of high stability.

A further object is to provide chelates or chelating agents suitable for labeling of biomolecules as such in solution.

Yet another object is to provide chelates suitable for labeling oligopeptides or oligonucleotides simultaneously with their synthesis on a solid phase.

Yet another object is to provide a solid support conjugated with chelates, chelating agents or biomolecules according to this invention.

Thus, according to one aspect this invention concerns a chelating agent comprising
- a chromophoric moiety,
- a chelating part comprising at least two carboxylic acid or phosphonic acid ester groups, attached to an aromatic unit of the chromophoric moiety, either directly or via an N-containing hydrocarbon chain, and
- a reactive group A, tethered to the chromophoric moiety, or to the chelating part via a linker- x, wherein
   (i) the chromophoric moiety comprises two or three pyridyl groups, wherein at least one of them is furylsubstituted and said pyridyl groups being tethered either directly to each other to form terpyridyl group or to each other via N-containing hydrocarbon chains,
   (ii) the linker x is formed from one to ten moieties, each moiety being selected from the group consisting of phenylene, alkylene containing 1-12 carbon atoms, ethynydiyl (-C≡C-), ethylenediyl (-C=C-)ether (-O-), thioether (-S-), amide (-CO-NH-, -CO-NR'-, NH-CO and -NR'-CO-), carbonyl (-CO-), ester (-COO- and -OOC-), disulfide (-SS-), diaza (-N=N-), and tertiary amine, wherein R' represents an alkyl group containing less than 5 carbon atoms,
   (iii) A is an amino acid residue -GH(NHR₁)R₅ where R₁ is a transient protecting group and R₅ is a carboxylic acid or its salt, acid halide or an ester.

According to another aspect, the invention concerns a chelate comprising
- a lanthanide ion,
- a chromophoric moiety,
- a chelating part comprising at least two carboxylic acid or phosphonic acid groups, or esters or salts of said acids, attached to an aromatic unit of the chromophoric moiety, either directly or via an N-containing hydrocarbon chain, and
- a reactive group A, tethered to the chromophoric moiety or to the chelating part via a linker x, wherein
   (i) the chromophoric moiety comprises two or three pyridyl groups, wherein at least one of them is furylsubstituted and said pyridyl groups either being tethered directly to each other to form terpyridyl group or to cach other via N-containing hydrocarbon chains,
   (ii) the linker x is formed from one to ten moieties, each moiety being selected from the group consisting of phenylene, alkylene containing 1-12 carbon atoms, ethynyldiyl (-C≡C-), ethylenediyl (-C=C-), ether (-O-), thioether (-S-), amide (-CO-NH-, -CO-NR'-, NH-CO and -NR'-CO-), carbonyl (-CO-), ester (-COO- and -OOC-), disulfide (-SS-), diaza (-N=N-), and tertiary amine, wherein R' represents an alkyl group containing less than 5 carbon atoms,
   (iii) the reactive group A is selected from the group consisting of isothiocyanate, haloacetamido, maleimido, dichlorotriazinyl, dichlorotriazinylamino, pyridyldithio, thioester, aminooxy, hydrazide, amino, a polymerizing group, and a carboxylic acid or acid halide or an active ester thereof.

According to a third aspect, the invention concerns a biomolecule conjugated with a chelate according to this invention.

According to a fourth aspect, the invention concerns a biomolecule conjugated with a chelating agent according to this invention.

According to a fifth aspect, the invention concerns a solid support conjugated with a chelate or a labeled biomolecule according to this invention.

According to a sixth aspect, this invention concerns a labeled oligopeptide, obtained by synthesis on a solid phase, by introduction of an appropriate chelating agent according to this invention into the oligopeptide structure on an oligopeptide synthesizer, followed by deprotection and optionally also introduction of a metal ion.

According to a seventh aspect, this invention concerns a labeled oligonucleotide, obtained by synthesis on a solid phase, by introduction of an appropriate chelating agent according to this invention into the oligonucleotide structure on an oligonucleotide synthesizer, followed by deprotection and optionally also introduction of a metal ion.

According to an eighth aspect, this invention concerns a solid support conjugated with the chelating agent according to claim 7, suitable for use in the synthesis of an oligonucleotide, wherein the reactive group A is

-Y-O-x'-

where
x' is a linker connected to a solid support, and is the same or different as the linker x Y is absent or is a radical of a purine or pyrimidine or any other modified base suitable for use in the synthesis of modified oligonucleotides, said base being connected to the oxygen atom via either
i) a hydrocarbon chain, which is substituted with a protected hydroxyethyl group, or via
ii) a furan ring or pyrane ring or any modified furan or pyrane ring, suitable for use in the synthesis of modified oligonucleotides.

Finally, the invention concerns also a chelate comprising the aforementioned chelating agent and a metal ion.

### DETAILED DESCRIPTION OF THE INVENTION

### Chelating agents

Chelating agents and metal chelates based thereon where the chromophoric moiety, which most commonly is a bivalent aromatic structure comprising one or more furylsubstituted pyridyl groups, are new. The furylsubstituted pyridyl group is capable of absorbing light or energy and transferring the excitation energy to the chelated lanthanide ion, giving rise to a strong fluorescense irrespective of the lanthanide ion used. In addition to the furylsubstituted pyridyl group or groups, the chromophoric unit may comprise unsubstituted pyridyl groups, pyridyl groups bearing other substituents and/or other aromatic groups.

The furyl group can be attached to pyridine ring via its C2 atom, or via its C3 atom by using 3-(tributylstannyl)furan instead of 2-(tributylstannyl)furan as the reagent in the synthesis strategy. In the compounds demonstrated by specific examples herein, the 4-position of the pyridyl group bears the furyl substituent. Although this position is believed to be the most preferable, other positions of the pyridine ring may also be useful for substitution.

The chromophoric moiety comprises two or three pyridyl groups, wherein at least one of them is furylsubstituted. These pyridyl groups can be tethered directly to each other to form a bipyridyl or terpyridyl group, respectively. Alternatively, and more preferably, the pyridyl groups are tethered to each other via N-containing hydrocarbon chains. In this case chelates with very good stability can be obtained.

Compounds comprising three aromatic units (such as three pyridyl groups wherein at least one of them is furyl substituted), e.g. compounds based on aza-crowns, are especially suitable in assays based on energy transfer or quenching, because in their emission spectra peak near 615 nm is more dominant and there is less disturbance in the wavelength used in the assays.

In case the chelating part is attached to the aromatic unit of the chromophoric moiety, it can be attached to the pyridine ring or to a substituent thereon such as the furyl group.

The chelating agent or chelate must bear a reactive group A in order to enable covalent binding of the chelating agent or chelate to a biomolecule or to a solid support. However, there exist applications where no such covalent binding is necessary. Chelating compounds of this invention can also be used in applications where no reactive groups in the chelate are needed. One example of this kind of technology is demonstrated e.g. in Blomberg, et al., J. Immunological Methods, 1996, 193, 199. Another example where no reactive group A is needed is the separation of eosinophilic and basophilic cells. In this application positively and negatively charged chelates bind negatively and positively charged cell surfaces, respectively.

Although that a reactive group A in principle in many applications could be attached directly to the chromophoric group or to the chelating part, it is highly desirable, especially for steric reasons, to have a linker x between the reactive group A and the chromophoric group or chelating part, respectively. The linker is especially important in case the chelate shall be used in solid phase syntheses of oligopeptides and oligonucleotides, but it is desirable also in labelling biomolecules in solution.

The reactive group A is selected from the group consisting of isothiocyanate, haloacetamido, maleimido, dichlorotriazinyl, dichlorotriazinylamino, pyridyldithio, thioester, aminooxy, hydrazide, amino, a polymerizing group, and a carboxylic acid or acid halide or an active ester thereof. Particularly in case the chelate or chelating agent shall be attached to microparticle or nanoparticle it is preferable to have a reactive group which is a polymerizing group. In this case the label can be introduced in the particle during the manufacturing of the particles.

The linker x is preferably formed from one to ten moieties, each moiety being selected from the group consisting of phenylene, alkylene containing 1-12 carbon atoms, ethynydiyl (-C≡C-), ethylenediyl (-C=C-), ether (-O-), thioether (-S-), amide (-CO-NH-, -CO-NR'-, NH-CO and -NR'-CO-), carbonyl (-CO-), ester (-COO- and -OOC-), disulfide (-SS-), diaza (-N=N-), and tertiary amine, wherein R' represents an alkyl group containing less than 5 carbon atoms.

The group A-x- can be tethered to the molecule in different ways. It can be tethered to the chelating part, to the N-containing chain joining the aromatic units together, or to an aromatic unit. In the last mentioned case it is preferable to tether the group A-x- to a furyl substituent. Such compounds are easy to prepare and they have been found to be very workable.

According to a particularly preferable embodiment, the chelating agent is one of the following specific structures:

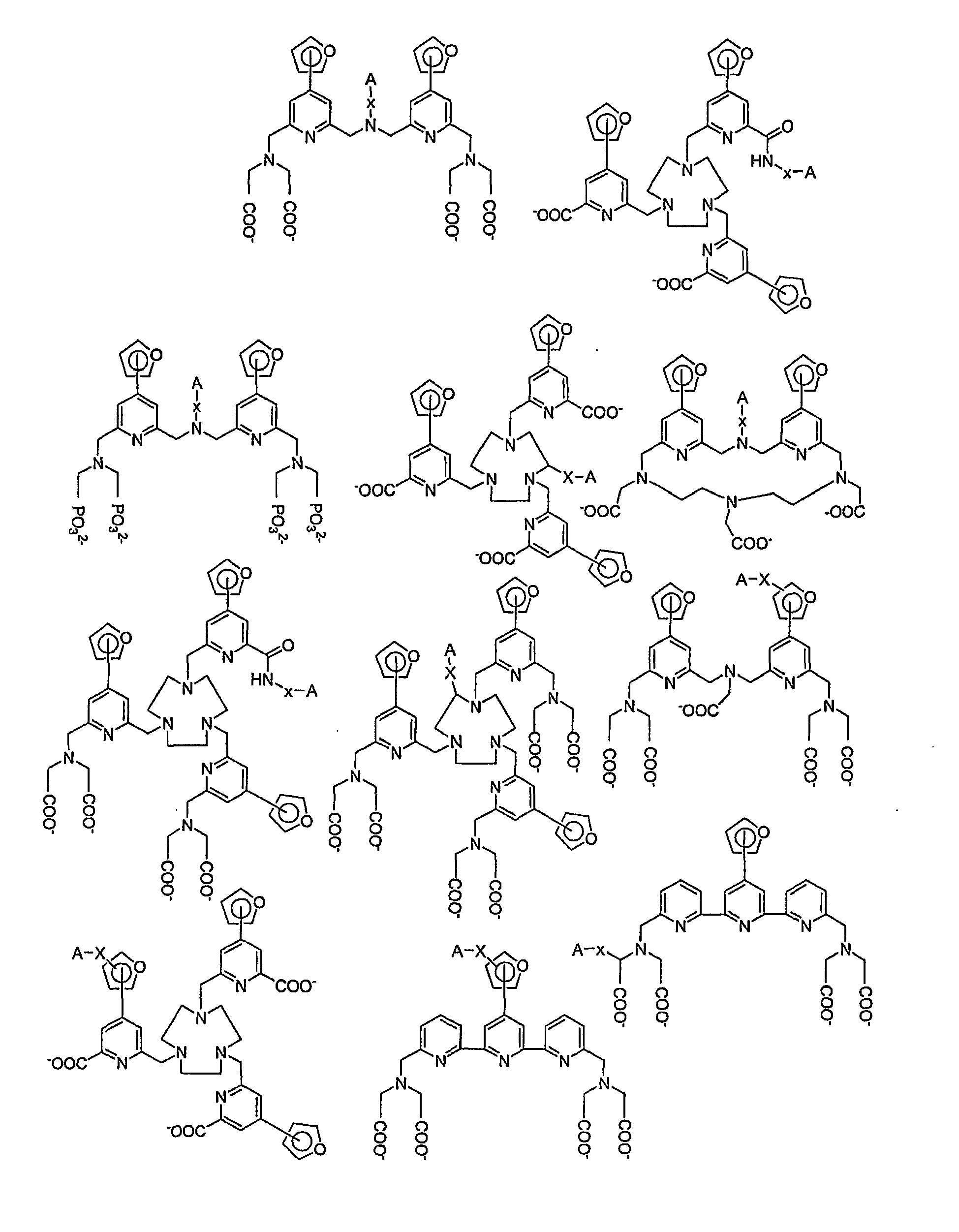

### Chelating agents for use in peptide synthesis

According to one preferred embodiment, the chelating agent according to this invention is suitable for use in the synthesis of an oligopeptide. In this application, the reactive group A is connected to the chelating agent via a linker x, and A is an amino acid residue -CH(NHR¹)R⁵ where R¹ is a transient protecting group and R₅ is a carboxylic acid or its salt, acid halide or an ester. Particularly preferable chelating agents are the structures 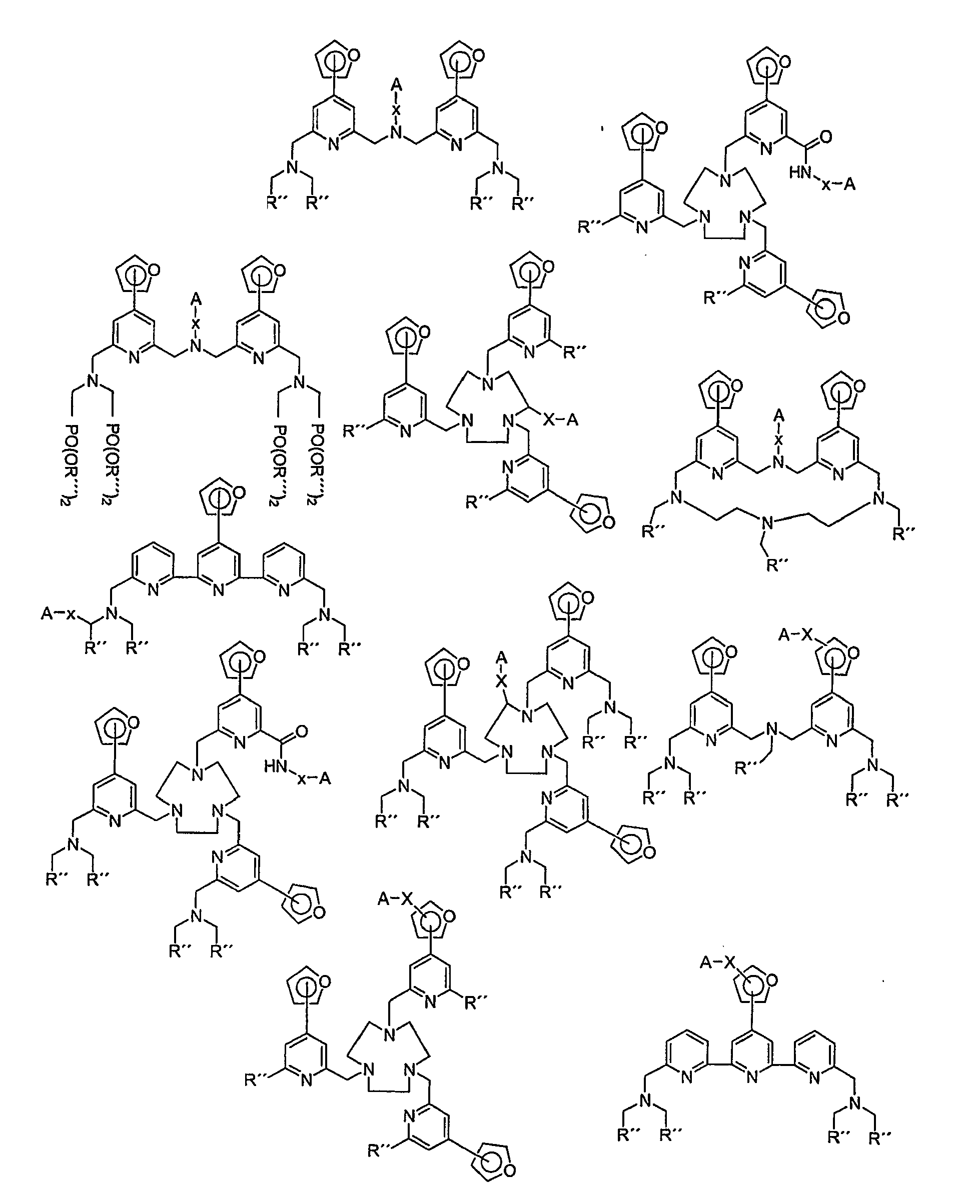 wherein x is as defined before and the protecting group R¹ is selected from a group consisting of Fmoc (fluorenylmethoxycarbonyl), Boc (*tert*-butyloxycarbonyl); or Bsmoc (1,1-dioxobenzo[b]thiophen-2-ylmethyloxycarbonyl), and R" is an alkyl ester or an allyl ester and R''' is an alkyl group.

The chelating agent can be introduced into biomolecules with the aid of peptide synthesizer. The chelating agent can be coupled to an amino tethered solid support or immobilized amino acid e.g. by carbodiimide chemistry described in Jones, J., The Chemical Synthesis of Peptides, Oxford Univesity Press, Oxford, 1994, (i.e. the carboxylic acid function of the labeling reagent reacts with the amino group of the solid support or amino acid in the presence of an activator). When the condensation step is completed the transient amino protecting group of the labeling reagent is selectively removed while the material is still attached to the solid support (e.g with piperidine in the case of Fmoc-protecting group). Then second coupling of a chelating agent or other reagent (amino acid, hapten) is performed as above. When the synthesis of the desired molecule is completed, the material is detached from the solid support and deprotected. Purification can be performed by HPLC techniques. Finally the purified ligand is converted to the corresponding metal chelate by addition of known amount of metal ion.

### Chelating agents for use in oligonucleotide synthesis

According to another preferred embodiment, the chelating agent according to this invention is suitable for use in the synthesis of an oligonucleotide. In this case the reactive group A is connected to the chelating agent via a linker x, and A is

-Y-O-PZ-O-R⁴

where
one of the oxygen atoms optionally is replaced by sulfur, Z is chloro or NR²R³, R⁴ is a protecting group, R² and R³ are alkyl groups, and Y is absent or is a radical of a purine base or a pyrimidine base or any other modified base suitable for use in the synthesis of modified oligonucleotides. Said base is connected to the oxygen atom either via i) a hydrocarbon chain, which is substituted with a protected hydroxyethyl group, or via ii) a furan ring or pyrane ring or any modified furan or pyrane ring, suitable for use in the synthesis of modified oligonucleotides.

The chelating agent can be introduced into oligonucleotides with the aid of oligonucleotide synthesizer. A useful method, based on a Mitsonobu alkylation (J Org Chem, 1999, 64, 5083; Nucleosides, Nucleotides, 1999, 18, 1339) is disclosed in EP-A- 1152010. Said patent publication discloses a method for direct attachment of a desired number of conjugate groups to the oligonucleotide structure during chain assembly. Thus solution phase labeling and laborious purification procedures are avoided. The key reaction in the synthesis strategy towards nucleosidic oligonucleotide building blocks is the aforementioned Mitsunobu alkylation which allows introduction of various chelating agents to the nucleoside, and finally to the oligonucleotide structure. The chelating agents are introduced during the chain assembly. Conversion to the lanthanide chelate takes place after the synthesis during the deprotection steps.

Normal, unmodified oligonucleotides have low stability under physiological conditions because of its degradation by enzymes present in the living cell. It may therefore desirable to create a modified oligonucleotide according to known methods so as to enhance its stability against chemical and enzymatic degradation. Modifications of oligonucleotides are extensively disclosed in prior art. Reference is made to US 5,612,215. It is known that removal or replacement of the 2'-OH group from the ribose unit in an RNA chain gives a better stability. WO 92/07065 and US 5,672,695 discloses the replacement of the ribose 2'-OH group with halo, amino, azido or sulfliydryl groups. US 5,334,711 discloses the replacement of hydrogen in the 2'-OH group by alkyl or alkenyl, preferably methyl or allyl groups. Furthermore, the intemucleotidic phosphodiester linkage can, for example, be modified so that one ore more oxygen is replaced by sulfur, amino, alkyl or alkoxy groups. Preferable modification in the internucleotide linkages are phosphorothioate linkages. Also the base in the nucleotides can be modified.

Preferably Y is a radical of any of the bases thymine, uracil, adenosine, guanine or cytosine, and said base is connected to the oxygen atom via i) a hydrocarbon chain, which is substituted with a protected hydroxyethyl group, or via ii) a furan ring having a protected hydroxyethyl group in its 4-position and optionally a hydroxyl, protected hydroxyl or modified hydroxyl group in its 2-position.

Preferably a reactive group -Y-O-P(NR²R³)-O-R⁴ has a structure selected from one of the following structures: 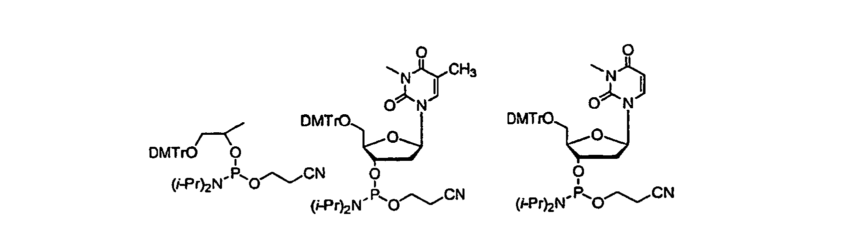 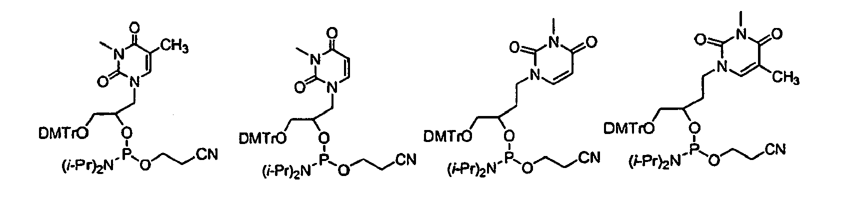 where - is the position of the linker x and DMTr is dimethoxytrityl.

A particularly preferable chelating agent for this use is selected from one of the specific structures disclosed below 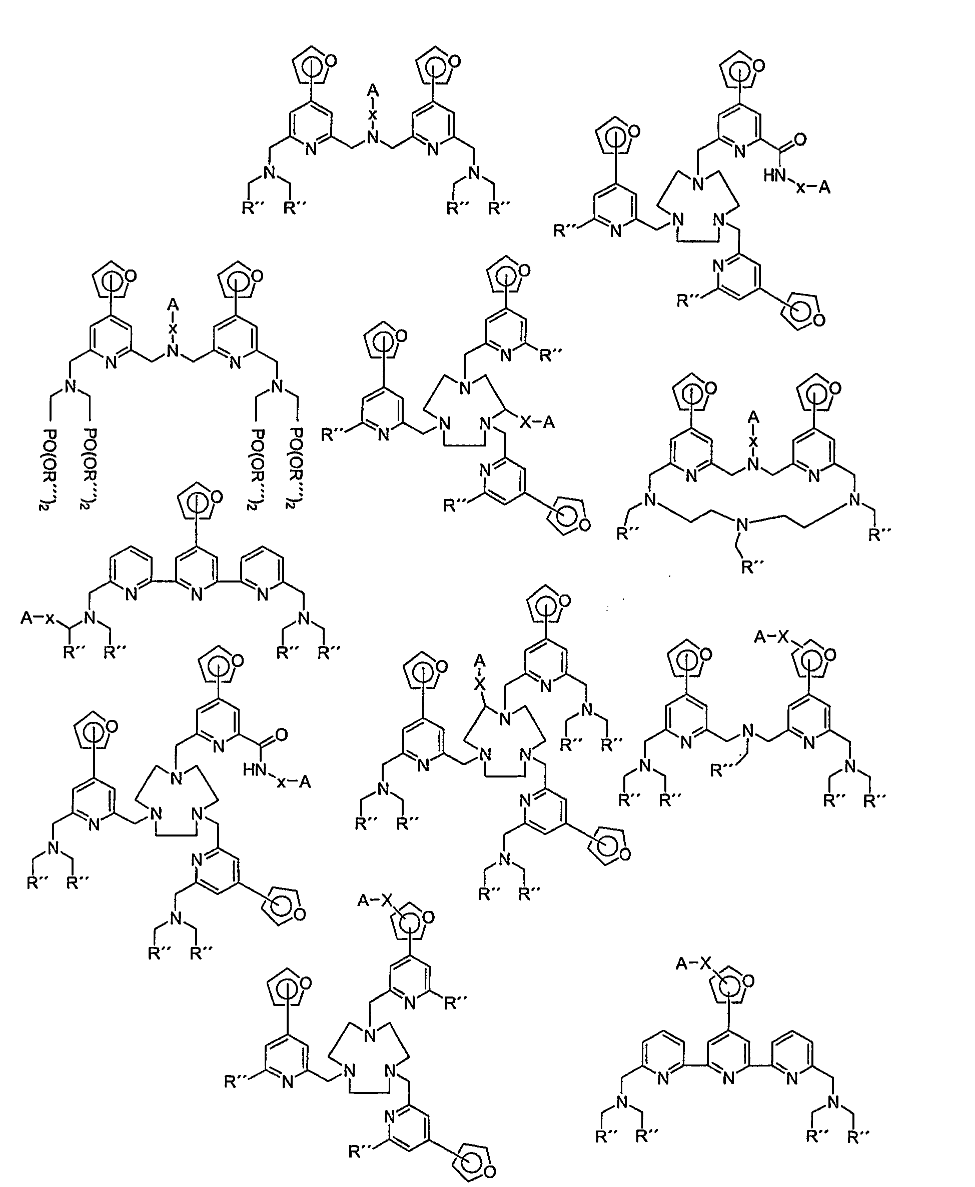 where R" is an alkyl ester or an allyl ester and R''' is an alkyl group and wherein x is as defined before and A is -Y-O-P(NR²R³)-O-R⁴ as defined above.

### Chelates

The chelates comprise a chelating agent as describes above and a chelated metal ion.

In case the chelate is to be used in bioaffinity assays, the chelated metal ion M is preferably a lanthanide, especially europium(III), samarium(III), terbium(III) or dysprosium(III). The chelating agent is preferably one of the preferable agents mentioned above.

Particularly preferable lanthanide chelates are 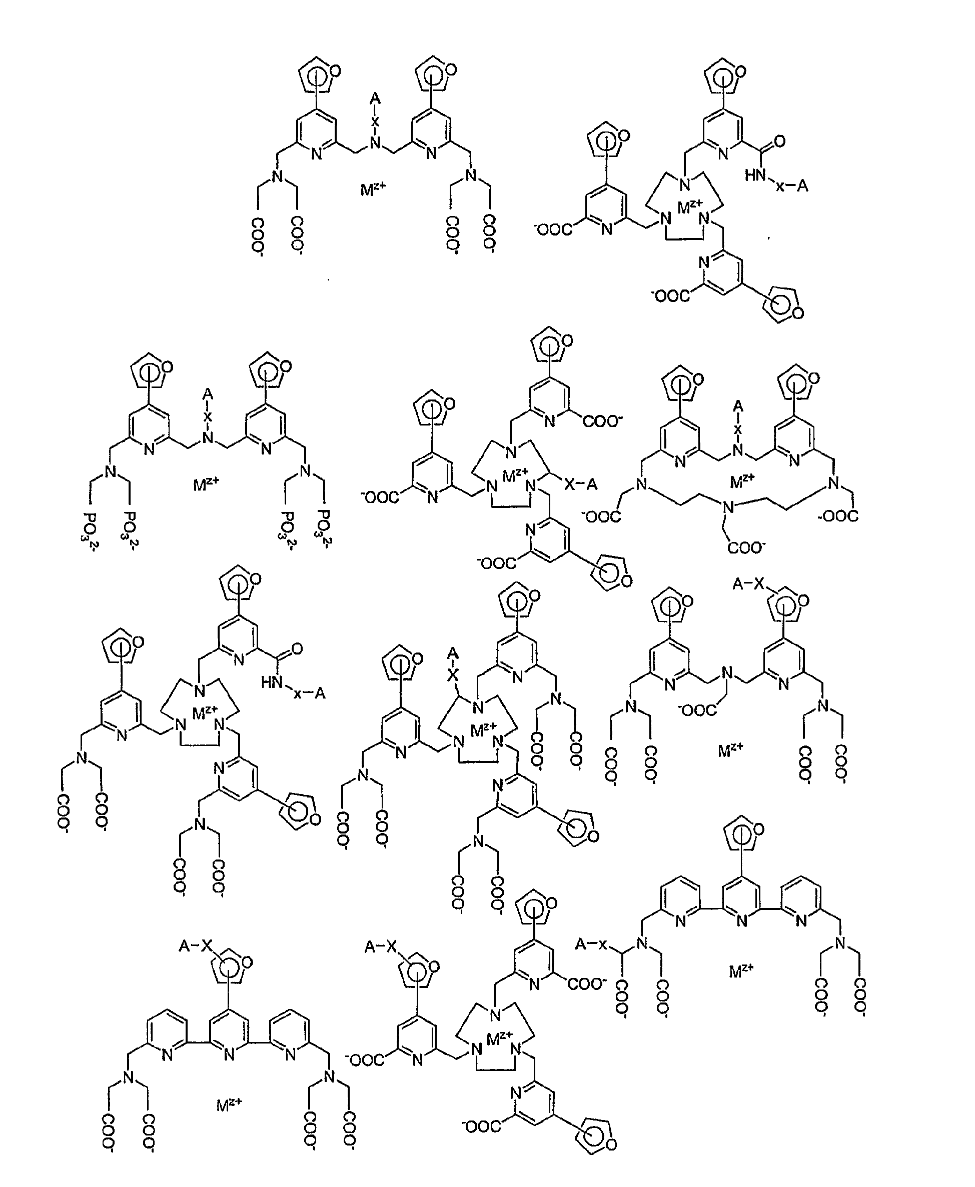 where M is a metal and z is 2 or 3.

In order to obtain very stable chelates, it is preferable to have a chromophoric moiety where there are several pyridyl groups tethered to each other via N-containing hydrocarbon chains.

### Biomolecules

The biomolecule conjugated with a chelating agent or a chelate according to this invention is preferably an oligopeptide, oligonucleotide, DNA, RNA, modified oligo- or polynucleotide, such as phosphoromonothioate, phosphorodithioate, phosphoroamidate and/or sugar- or basemodified oligo- or polynucleotide, protein, oligosaccaride, polysaccaride, phospholipide, PNA, LNA, antibody, hapten, drug, receptor binding ligand and lectine.

### Solid support conjugates

The chelates, chelating agents and biomolecules according to this invention may be conjugated on a solid support. The solid support is preferably a particle such as a microparticle or nanoparticle, a slide or a plate.

In case the chelate or chelating agent has a polymerizing group as reactive group, then the chelate or chelating agent may be introduced in the solid support, for example a particle, simultaneously with the preparation of the particles.

The biomolecule conjugated with the solid support, either covalently or noncovalently is preferable a labeled oligopeptide, obtained by synthesis on a solid phase, by introduction of a chelating agent into the oligopeptide structure on an oligopeptide synthesizer, followed by deprotection and optionally introduction of a metal ion. Alternatively, the biomolecule conjugated with the solid support, either covalently or noncovalently is preferable a labeled oligonucleotide, obtained by synthesis on a solid phase, by introduction of a chelating agent into the oligonucleotide structure on an oligonucleotide synthesizer, followed by deprotection and optionally introduction of a metal ion.

A solid support conjugated with a chelating agent having a reactive group A which is connected to the chelating agent via a linker x, and A is -Y-O-x'- as defined before, is suitable for use in oligonucleotide syntheses.

The invention will be illuminated by the following non-restrictive Examples.

### EXAMPLES

The invention is further elucidated by the following examples. The structures and synthetic routes employed in the experimental part are depicted in Schemes 1-11. Scheme **1** illustrates the synthesis of the chelates **3.** The experimental details are given in Examples 1-3. Scheme 2 illustrates the synthesis of the chelates **9**. Experimental details are given in Examples 4-9. Scheme 3 illustrates the synthesis of the labeling reagents **13.** Experimental details are given in Examples 10-13. Scheme 4 illustrates the synthesis of the building block **18** designed for the introduction of lanthanide chelates to the oligopeptide structure on solid phase. Experimental details are given in Examples 14-18. Scheme 5 illustrates the synthesis of the labeling reagents **21-23.** Experimental details are given in Examples 19-23. Scheme 6A and B illustrates the synthesis of the labeling reagents **34.** Experimental details are given in Examples 24-34. Scheme 7 illustrates the preparation of oligonucleotide labeling reagents for the solid phase introduction of lanthanide chelates to the oligonucleotide structure on solid phase. Scheme 8 illustrates the synthesis of a chelate where the furan moiety is linked to the pyridine ring via C3. Scheme 9 illustrates the labeling of a hapten with the labeling reagent **23.** Experimental details are given in Example 37. Scheme 10 illustrates the labeling of an oligopeptide on solid phase using block 18. Experimental details are given in Example 35. Scheme 11 illustrates the labeling of an oligopeptide in solution using labeling reactant **34a**. Experimental details are given in Example 36. Scheme 12A illustrates the Examples 38 to 45, Scheme 12B illustrates the Examples 46 to 47, and Scheme 13 illustrates the Examples 48 to 54.

### Experimental procedures

Reagents for machine assisted oligopeptide synthesis were purchased from Applied Biosystems (Foster City, CA). Adsorption column chromatography was performed on columns packed with silica gel 60 (Merck). NMR spectra were recorded either on a Brucker 250 or a Jeol LA-400 spectrometers operating at 250.13 and 399.8 MHz for ¹H, respectively. Me₄Si was used as an internal reference. Coupling constants are given in Hz. IR spectra were recorded on a Perkin Elmer 2000 FT-IR spectrophotometer. Fast atom bombardment mass spectra were recorded on a VG ZabSpec-ao TOF instrument in the positive detection mode. Electrospray mass spectra were recorded on a Applied Biosystems Mariner ESI-TOF instrument. Oligopeptides were assembled on an Applied Biosystems 433A Synthesizer using recommended protocols. Fluorescence spectra were recorded on a PerkinElmer LS 55 instrument.

### Example 1

### The synthesis of 2,2',2",2'''-{[4-(2-furyl)pyridine-2,6-diyl)]bis(methylenenitrilo)}tetrakis(acetic acid) tetra(tert-butyl ester) 1

2,2',2",2'''-{[4-bromopyridine-2,6-diyl)]bis(methylenenitrilo)}tetrakis(acetic acid) tetra(*tert*-butyl ester) (0.29 g, 0.43 mmol) and 2-(tributylstannyl)furan (0.15 g, 0.43 mmol) were dissolved in DMF (2.0 mL) and deaerated with argon. Tetrakis(triphenylphosphine)palladium(0) (0.025 g, 0.020 mmol) was added, and the mixture was stirred at 100 °C for 5 h. The mixture was cooled to room temperature and concentrated in vacuo. Purification was performed on silica gel (eluent: initially petroleum ether, then petroleum ether/ethyl acetate 5:2 (v/v) Yield was 0.14 g (50 %). ¹H NMR (400 MHz, CDCl₃): 1.46 (36H, s); 3.51 (8H, s); 4.06 (4H, s); 6.48-6.53 (1H, m); 6.92-6.97 (1H, m); 7.50-7.52 (1H, m); 7.74-7.78 (2H, m); MS: 682 (MH⁺);. UV λₘₐₓ (H₂O) 290, **227 IR** (film)/cm⁻¹ 1782 (C=O), 1146 (C-O).

### Example 2

### The synthesis of 2,2',2",2'''- {4-(2-furyl)pyridine-2,6-diyl]bis(methylenenitrilo)}tetrakis(acetic acid), 2

Compound **1** (20 mg, 0.03 mmol) was dissolved in trifluoroacetic acid (0.5 mL) and stirred for 3.5 h at room temperature. All volatile materials were removed in vacuo, and the residue was triturated with diethyl ether. The precipitation was colledted by filtration and dried. Yield was 9.3 mg (46%). ¹H NMR (400 MHz, DMSO-*d₆*): d 3.60 (8H, s); 4.19 (4H, s); 6.79-6.82 (1H, m); 7.42-7.48 (1H, m); 7.95-7.99 (1H, m); 8.03-8.06 (1H, m); ). MS 436 (M⁺). UV λₘₐₓ/nm (H₂O) 301. IR (film)/cm⁻¹ 1735; 1618 (C=O), 1195 (C-O).

### Example 3

### The synthesis of of 2,2',2",2'''-{4-(2-furyl)pyridine-2,6-diyl]bis(methylenenitrilo)}tetrakis(acetic acid) lanthanide(III) 3.

Compound **2** was converted to the corresponding europium(III) **3a** and samarium(III) chelates (**3b**) as described in Takalo et al, Bioconjugate Chem., 1994, 5, 278.

### Example 4

### The synthesis of 4'-(2-furyl)-2,2':6',2"-terpyridine 4

4'-(trifluoromethanesulfonato)-2,2':6'-2 "-terpyridine (2.0 g, 5.2 mmol) was dissolved in dry DMF (10 mL). Dry TEA (2.3g, 23 mmol) and 2-(tributylstannyl)furan (2.6 g, 7.3 mmol) were added and the mixture was deaerated with agron. Dichlorobis(triphenylphosphine)palladium(II) (0.10 g, 0.15 mmol) was added, and f the mixture was heated at 90 °C for 1 h under argon atmosphere. Water (200 mL) was added and stirring was continued for 1h at room temperature. The precipitate formed was filterered and washed with could water (3 · 20 mL). The precipitate was suspended in diethyl ether (120 mL), filtered and washed with diethyl ether (3 · 30 mL). Purification on silica gel (eluent: chloroform:methanol:ammonium hydroxide 12:1:0.25 v/v/v). Yield was 1.4 g (88%). ¹H NMR (400 MHz, CDCl₃): δ 6.57 (1H, dd, *J* 2 and 4); 7.12 (1H, d, *J*4); 7.36 (2H, ddd, *J* 1, 5 and 8); 7.59 (1H, d, J2); 7.87 (2H, dt, *J* 2 and 8); 8.65 (2H, br d, *J* 8); 8.72 (2H, s); 8.74 (2H, br d, *J* 5); MS 300 [M⁺]. UV λₘₐₓ/nm (EtOH) 268, 251.

### Example 5

### The synthesis of 4'-(furyl)-2,2':6',2"-terpyridine-N,N"-dioxide 5

Compound **4** (0.15 g, 0.50 mmol) was dissolved in dichloromethane (5.0 mL). 3-chloroperbenzoic acid (0.33 g, 1.9 mmol) was added portionwise, and the mixture was stirred for 6 h at room temperature. Solvent was evaporated off in vacuo, and the crude product was purified on silica gel (eluent: dichloromethane:methanol, 95:5 (v/v)). Yield was 83 mg (50%). ¹H NMR (400 MHz, CDCl₃): δ 6.55 (1H, m); 7.10 (1H, m); 7.32 (2H, m); 7.41 (1H, m); 7.58 (1H, m); 8.22 (2H, br d, *J*8); 8.40; 2H, d, *J* 6); 9.19 (2H, s); MS 332 [M⁺]. UV λₘₐₓ/nm (EtOH) 288, 249, 234. IR(film) 1269 cm⁻¹ (N-O).

### Example 6

### The synthesis of 4'-(2-furyl)-2,2':6',2"-terpyridine-6,6"-dicarbonitrile 6

Compound 5 (0.070 g, 0.21 mmol) was dissolved in dichloromethane (5.0 mL). Trimethylsilylcyanide (0.21 g, 2.1 mmol) was added, and the mixture was stirred for 5 min, followed by dropwice addition of benzoyl chloride (0.12 g, 0.84 mmol). The reaction was allowed to proceed for 1.5 h at room temperature. Potassium carbonate (10-% w/v in water; 5.0 mL) was added, and the mixture was stirred for 1.5 h at room temperature. The precipitate formed was filtered and washed with water (2 · 10 mL) and dichloromethane (2 · 10 mL). Yield was 0.024 g (33 %). MS 530 [M⁺]. IR (KBr)/cm⁻¹: 2286 (C≡N); 1610 (C-N).

### Example 7

### The synthesis of 2,2';2",2'''-{[4'-(2-furyl)-2,2':6',2"-terpyridine-6,6'-diyl]bis(methylenenitrilo)}tetrakis(acetate) tetra-tert-butyl ester, 7

Compound **6** (22 mg), 0.063 mmol) was suspended ibn dry THF (1.5 mL) and deaerated with argon. Borane-THF complex (1M, 23 mg, 0.27 mmol) was added dropwice during 5 min, and the mixture was stirred for 16 h at room temerature. The reaction was quenced by addition of dry methanol (4.0 mL). All volatile materials were removed in vacuo, and the residue was dissolved in methanolic HCl (10 mL) and strirred for 1 h at rt and concentrated. The residue was suspended in dry THF and filtered and dried. The residue was dissolved in dry DMF (3.0 mL) and deaerated with argon. DIPEA (66 mg, 0.51 mmol), *t*-butyl bromoacetate (60 mg, 0.31 mmol) and potassium iodide (7.0 mg, 0.043 mmol) were added and the mixture was stirred for 16 h at room temperature. All volatile materials were removed in vacuo. Purification was performed on silica gel (eluent: petroleum ether: ethyl acetate: TEA; 10:1:1, v/v/v). yield was 15 mg. ¹H NMR (400 MHz, CDCl₃): δ 1.49 (36H, s); 4.19 (4H, s); 6.57 (1H, m); 7.12 (1H, m); 7.57 (1H, m); 7.70 (2H, d, *J* 7.59); 7.85 (2H, t, *J*7.5); 8.51 (2H, d, *J* 7.5); 8.72 (2H, s). MS 836 [M⁺]. UV γₘₐₓ/nm (EtOH) 289, 253IR (film)/cm⁻¹: 1733 (C=O); 1145 (C-O-O).

### Example 8

### The synthesis of 2,2';2",2'''-{[4'-(2-furyl)-2,2':6',2"-terpyridine-6,6'-diyl]bis(methylenenitrilo)}tetrakis(acetic acid), 8

Compound **7** (4.1 mg, 0.0051 mmol) was dissolved in TFA (1.0 mL), and the mixture was stirred for 4 h at room temperature and concertated. The residue was truiturated with diethyl ether, collected by filtration and dried. Yield was 3.6 mg (86%). ¹H NMR (400 MHz, CDCl₃): δ 3.59 (8H, s); 4.14 (4H, s); 6.76 (1H, dd, *J* 1.8 and 3.5); 7.42 (1H, d, *J*3.5); 7.66 (1H, d, *J* 8.0); 7.98 (1H, s); 8.02 (2H, t, *J* 8.0); 8.52 (2H, d, *J* 8.0); 8.67 (2H, s). UV γₘₐₓ/nm (EtOH) 289, IR (film)/cm⁻¹: 1685 (C=O); 1200 (C-O-O).

### Example 9

### The synthesis of 2,2'-2",2'''-{[4'-(2-furyl)-2,2':6',2"-terpyridine-6,6'-diyl]bis(methylenenitrilo)}tetralcis(acetic acid) lanthanide(III), 9

Compound 8 was converted to the corresponding europium(III) **9a** and samarium(III) chelates **9b** as described in Example 3.

### Example 10

### The synthesis of 2,2',2",2'''-{[2-(4-aminophenyl)ethylimino]bis(methylene)bis[4-(2-furyl)pyridine-6,2-diyl)]bis(methylenenitrilo)}tetrakis(acetic acid) tetra(tert-butyl ester), 10

2,2',2",2'''-{[2-(4-aminophenyl)ethylimino]bis(methylene)bis(4-bromopyridine-6,2-diyl) bis(methylenenitrilo)}tetrakis(acetic acid) tetra(*tert*-butyl ester) (0.30 g, 0.30 mmol) was allowed to react with and 2-tributylstannylfuran (0.22 g, 0.60 mmol) as described in Example 1. Yield was 0.22 g (75%). ¹H NMR (400 MHz, CDCl₃): δ 1.45 (36H, s); 3.50 (8H, s); 3.88 (4H, s); 4.04 (4H, s); 6.50 (1H, s); 6.87 (1H, s); 8.02 (4H, s). MS 965 [M⁺]. UV γₘₐₓ/nm (EtOH) 287, 226. IR (film) / cm⁻¹: 1735 (C=O), 1149 (C-O).

### Example 11

### Synthesis of 2,2',2",2"'-{[2-(4-aminophenyl)ethylimino]bis(methylene)bis[4-(2-furyl)pyridine-6,2-diyl)]bis(methylenenitrilo)}tetrakis(acetic acid), 11

Deprotection of compound **10** with TFA as described in Example 2 yielded the title compound. Yield was 70% ¹H NMR (400 MHz, DMSO-*d₆*): δ 3.56 (8H, s); 4.08 (4H, s); 6.73 (2H, m); 6.90 (2H, m); 7.13 (2H, m); 7.21 (2H, m); 7.73 (2H, s); 7.90 (2H, s); 7.92 (2H, s). MS 853 [M⁺]. UV γₘₐₓ/nm (H₂O) 303. IR (film) / cm⁻¹: 1674(C=O), 1617 (C=O), 1200 (C-O).

### Example 12

### The synthesis of 2,2',2",2'''-{[2-(4-aminophenyl)ethylimino]bis(methylene)bis[4-(2-furyl)pyridine-6,2-diyl)]bis(methylenenitrilo)}tetrakis(acetic acid) lanthanide (III), 12

Compound **11** was converted to the corresponding europium(III) **12a** and samarium(III) chelates **12b** as described in Example 3.

### Example 13

### The synthesis of 2,2',2",2'''-{[2-(4-isothiocyanatophenyl)ethylimino]bis(methylene)bis[4-(2-furyl)pyridine-6,2-diyl)]bis(methylenenitrilo)}tetrakis(acetic acid) lanthanide (III), 13

Compounds **12** were converted to the corresponding isothiocyanato derivatives as described in Takalo et al, Bioconjugate Chem., 1994, 5, 278.

### Example 14

### The synthesis of 2,2',2",2'''-{[6-(-methoxytrityl)hexylimino]bis(methylene)bis(4-bromo)pyridine-6,2-diyl)bis(methylenenitrilo)}tetrakis(acetic acid) tetra(tert-butyl ester) 14.

[(4-bromo-6-bromomethyl-2-pyridyl)methylenenitrilo]bis(acetic acid) di(*tert*-butyl ester) (2.06 g, 6.0 mmol) and 6-(4-methoxytrityl)hexanediamine (1.15 g, 3.2 mmol) were dissolved in dry DMF (100 ML) DIPEA (5.2 mL, 30 mmol) was added, and the mixture was stirred at rt for 2h and concentratred. Purification on silica gel yielded 1.30 g of compound **14.**

### Example 15

### The synthesis of 2,2',2,2'''-{[6-(4-methoxy)tritylaminohexyl]bis(methylene)bis[4-(2-furyl)pyridine-6,2-diyl)]bis(methylenenitrilo)}tetrakis(acetic acid) tetra(tert-butyl ester) 15.

Compound **14** (1.11 g, 0.91 mmol) and 2-(tributylstannyl)furan (0.68 g, 1.9 mmol) were dissolved in DMF (35 mL) and deaerated with argon.
Tetrakis(triphenylphosphine)palladium(0) (0.12 g) was added, and the mixture was stirred at 90 °C for 2.5 h. The mixture was cooled to room temperature and concentrated in vacuo. Purification was performed on silica gel (eluent: petroleum ether/ethyl acetate/ TEA 5:1:1 (v/v/v); then 5:3:1 (v/v/v)). Yield was 0.83 g (75%).

### Example 16

### The synthesis of 2,2',2",2'''-{[6-(aminohexyl]bis(methylene)bis[4-(2-furyl)pyridine-6,2-diyl)]bis(methylenenitrilo)}tetrakis(acetic acid) tetra(tert-butyl ester) 16.

Compound **15** (0.95 g, 0.80 mmol) was dissolved in the mixture of TFA (0.25 mL) and dichlromethane (25 mL) and the mixture was stirred for 2 h at rt. The reaction mixture was washed with sat. NaHCO₃. The organic phase was dried over Na₂CO₃, concetrated and purified on silica gel. Yield was 0.61 g (83%).

### Example 17

The synthesis of protected oligopeptide labeling reactant, **17**.

Compound **16** (0.57 g, 0.62 mmol) and Fmoc-Glu-Oall (0.3 g, 0.74 mmol) were dissolved in dichloromethane (25 mL). DCC (0.15 g, 0.74 mmol; predissolved in 3 mL of DCM) was added, and the reaction was allowed to proceed for 6 h at rt. The precipitation formed was filtered off, and the filtrate was concventrated and purified on silica gel.

### Example 18

The synthesis of the oligopeptide labeling reactant **18**.

Compound **17** (0.42 g, 0.32 mmol) was dissolved in DCM (10 mL) and deaerated with argon. Pd(PPh₃)₄ (8 mg, 0.007 mmol) and PhSiH₃ (69 mg, 0.64 mmol) were added, and the reaction was allowed to proceed for 30 min at rt, and washed with 10% citric acid. The organic layer was separated, dried over 4Å molecular sieves and concentrated.

### Example 19

### Synthesis of 2,2',2",2"'-{[6-aminohexyl]bis(methylene)bis[4-(2-furyl)pyridine-6,2-diyl)]bis(methylenenitrilo)}tetrakis(acetic acid), 19

The synthesis was performed as described in Example 2 for compound 3. Yield was 81%. ¹H NMR (400 MHz, DMSO-*d₆*): δ 1.25-1.32 (4H, m); 1.47-1.55 (2H, m); 1.72-1.81 (2H, m); 2.70-2.78 (2H, m); 3.18-3.23 (2H, m); 3.55 (8H, s); 4.06 (4H, s); 4.53 (4H, s); 6.73 (2H, dd, *J* 1.6 and 3.6) 7.20 (2H, d, *J* 3.6); 7.72 (2H, s); 7.93 (2H, d, *J* 1.6). MS 746 (M⁺). UV γₘₐₓ/nm (H₂O) 302. IR (KBr)/cm⁻¹ 1676; 1618 (C=O), 1199 (C-O).

### Example 20

### Synthesis of 2,2',2",2'''-{[6-aminohexylimino]bis(methylene)bis[4-(2-furyl)pyridine-6,2-diyl)]bis(methylenenitrilo)}tetrakis(acetic acid) lanthanide(III), 20.

Compound **19** (50 mg, 0.060 mmol) was converted to the corresponding europium(III) **20a** and samarium(III) **20b** as described in Example 3.

### Example 21

### Synthesis of 2,2',2",2'''-{[6-iodoacetamidohexylimino]bis(methylene)bis[4-(2-furyl)pyridine-6,2-diyl)]bis(methylenenitrilo)}tetrakis(acetic acid) lanthanide (III) 21.

Compound 20 was converted to the corresponding iodoacetamido derivatives **21** as described in Takalo et al, Bioconjugate Chem.,1994, 5, 278*.*

### Example 22

### The synthesis of 2,2',2",2'''- {[6-isothiocyanatohenylthioureido)hexylimino]bis(methylene)bis[4-(2-furyl)pyridine-6,2-diyl)]bis(methylenenitrilo)}tetrakis(acetic acid) lanthanide(III), 22.

Phenylene-1,4-diisothiocyanate (6.5 mg; 0.034 mmol) was dissolved in the mixture of pyridine:water:TEA (9:1.5:0.1, v/v/v; 0.5 mL). Compound **20** (15 mg, 0.017 mmol; predissolved in the same solution; 0.5 mL) was added dropwise, and f the reaction was allowed to proceed for 1 h at rt. Acetone (1.0 mL) was added, and the precipitation was centrifugated.

### Example 23

### The synthesis of 2,2',2",2"'-{[6-isothiocyanatohexylimino]bis(methylene)bis[4-(2-furyl)pyridine-6,2-diyl)]bis(methylenenitrilo)}tetrakis(acetic acid) lanthanide(III), 23.

Compound 20 (100 mg) was converted to the corresponding isothiocyanato derivatives as described in Takalo et al, Bioconjugate Chem., 1994, 5, 278.

### Example 24

### The synthesis of 4-(2-furyl)-pyridine-2,6-diacetate diethyl ester, 24

4-bromopyridine-2,6-dicarboxylate diethyl ester (3.70 g; 12.3 mmol) was allowed to react with 2-(tributylstannyl)furan (12.3 mmol) as described in Example 1. Yield was 3.49 g (8.6 %). ¹H NMR ¹H-NMR (CDCl₃): 8.47 (2H, s), 7.63 (1H, d, *J* 1.6), 7.08 (1H, d, *J* 3.6 Hz), 6.59 (1H, dd, *J* 1.9 and 3.5 Hz), 4.51 (4H, q, *J* 7.1), 1.48 ( 6H, t, *J* 7.1). ESI-TOF-MS mass for C₁₅H₁₆NO₅ (M+H)⁺: calcd, 290.10 ; found, 290.12.

### Example 25

### The synthesis of 4-(furan-2-yl)-6-(hydroxymethyl)pyridine-2-carboxylic acid ethyl ester 25

Compound **24** (3.30 g; 11.4 mmol) was suspended in ethanol (150 mL). The suspension was dissolved by heating to 35 °C. NaBH₄ (0.43 g; 11.4 mmol) was then added, and the mixture was stirred at RT for 40 min. Reaction was quenched by adjusting pH to 3 with aqueous 6M HCl solution. Solvents were evaporated and the residue suspended in water (100 mL) and pH was adjusted to 7 with NaHCO₃. Product was extracted from water with CH₂Cl₂: MeOH (1:1 v/v) solution. CH₂Cl₂-layer was washed twice with water and dried over MgSO₄. Purification was performed on silica gel (eluent: petroleum ether bp 40-60 °C: ethyl acetate: triethylamine 5:2:1 v/v/v ).Yield was 1.76 g (62.4 %). ¹H NMR (CDCl₃): δ 1.46 (3H, t, *J*7); 4.49 (2H, q, *J*7); 4.88 (2H, s); 6.57 (1H, dd); 7.00 (1H, d); 7.58 (1H, m); 7.73 (1H, m); 8.23 (1H, d). ESI-TOF-MS mass for C₁₃H₁₄NO₄ (M+H)⁺: calcd, 248.09; found, 248.10.

### Example 26

### The synthesis of 6-(bromomethyl)-4-(furan-2-yl)pyridine-2-carboxylic acid ethyl ester 26

PBr₃ (0.65 mL; 6.9 mmol) was added to DMF (50 mL) and the mixture was cooled on an ice bath until a white precipitation formed. Compound **25**(1.70 g, 6.9 mmol; predissolved in 50 mL of DMF) was added and the mixture was stirred at RT for 40 min. The mixture was neutralized with saturated NaHCO₃ and water (100 mL) was added. The product was extracted with CH₂Cl₂. Dichloromethane layer was dried over MgSO₄ and evaporated to dryness. Purification was performed on silica gel (eluent: 5% MeOH/CH₂Cl₂). Yield was 2.13 g (87 %). ESI-TOF-MS mass for C₁₃H₁₃BrNO₃ (M+H)⁺: calcd, 310.01; found, 310.01.

### Example 27

### The synthesis of {2-{[4-(furan-2-yl)-6-(hydroxymethyl)pyridine-2-carbonyl]amino} ethyl} carbamic acid 4-nitrobenzyl ester 27

Compound **25** (0.42 g; 1.7 mmol) was dissolved in ethylenediamine (10 ml). The mixture was stirred at RT for 3 hours and evaporated to dryness. The residue was reevaporated twice from toluene and then dissolved in THF (20ml). TEA (0.24 ml;1.7 mmol) was added and 4-nitrobenzylchloroformate (0.76 g; 3.4 mmol) in 10 ml THF was added dropwise. The mixture was stirred at RT for 2 hours. The precipitation was filtered off and the filtrate was evaporated o dryness. The residue was suspended to CH₂Cl₂ and product was filtered. Purification on silica gel (eluent: 3% MeOH/ CH₂Cl₂). Yield was 0.46 g (61.3 %). ¹H-NMR (CDCl₃): 8.21 (2H, d, *J* 8.8H), 8.13 (1H, s), 7.94 (1H, s), 7.87 (1H, s), 7.60 (2H, d, *J* 8.8), 7.42 (1H, d, *J* 3.4), 6.72 (1H, dd, *J* 1.7 and 3.4), 5.18 (2H, s), 4.66 (2H, d, *J* 5.6 Hz), 3.42 (2H, m), 3.23 (2H, m). ESI-TOF-MS mass for C₂₁H₂₁N₄O₇ (M+H)⁺: calcd, 441.14 ; found: 441.14.

### Example 28

### The synthesis of {2-{[6-(bromomethyl)-4-(furan-2-yl)pyridine-2-carbonyl]amino}ethyl}carbamic acid 4-nitrobenzyl ester 28

PBr₃ (0.10 ml; 1.0 mmol) was added to 10 ml DMF and the mixture was cooled on ice bath until white precipitation formed. DMF-solution (10 mL) of compound **27** (0.45 g; 1.0 mmol) was added and the mixture was stirred at RT for 1 hour. Mixture was neutralized with saturated NaHCO₃ and 20 ml water was added. The product was extracted with CH₂Cl₂. Dichloromethane layer was dried with Na₂SO₄ and evaporated to dryness. Purification on silica gel (eluent: 10% MeOH/CH₂Cl₂). Yield was 0.39 g ( 75.8%) ¹H-NMR (CDCl₃): 8.82 (1H, t, *J* 4.5), 8.20 (2H, d, *J* 9.2), 8.03 (1H, d, *J* 1.6), 7.90 (1H, d, *J* 1.6), 7.59 (2H, d, *J* 8.7), 7.46 (1H, d, *J* 3.2), 6.74 (1H, dd, *J* 1.6 and 3.5), 5.18 (2H, s), 4.76 (2H, s), 3.43 (2H, m), 3.25 (2H, m). ESI-TOF-MS mass for C₂₁H₂₀BrN₄O₆ (M+H)⁺: calcd, 503.06 ; found, 503.06.

### Example 29

### The synthesis of 7-{4-(furan-2-yl)-6-[2-(4-nitrobenzyloxycarbonylamino)ethylcarbamoyl]pyridin-2-ylmethyl}-[1,4,7]triazacyclononane-1,4-dicarboxylic acid di-tert-butyl ester 29

[1,4,7]triazacyclononane-1,4-dicarboxylic acid di-*tert*-butyl ester (0.75 g; 2.3 mmol) and Compound **28** (1.15 g; 2.3 mmol) were dissolved in dry DMF (60 mL). 2,0 ml of DIPEA (11.4 mmol) was added and the mixture was stirred at RT for overnight. Solvent was evaporated to dryness and product was purified on silica gel ( eluent: diethyl ether). Yield was 1.20 g ( 80.5 %). ESI-TOF-MS mass for C₃₇H₅₀N₇O₁₀ (M+H)⁺: calcd, 752.36; found, 752.40.

### Example 30

### The synthesis of {2-{[4-(furan-2-yl)-6-([1,4,7]triazacyclononanyl-1-methyl)pyridine-2-carbonyl]amino}ethyl}carbamic acid 4-nitrobenzyl ester 30

Compound **29** (1.0 g; 1.3 mmol) was dissolved in 25 ml TFA and mixture was stirred at RT for 30 min. Solvent was evaporated to dryness. ESI-TOF-MS mass for C₂₇H₃₄N₇O₆ (M+H)⁺: calcd, 552.26; found, 552.26.

### Example 31

### The synthesis of {2-{{4-(furan-2-yl)-6-{4,7-bis[2-ethoxycarbonyl-4-(furan-2-yl)pyridin-2-ylmethyl]-[1,4,7]triazacyclononanyl-1-methylpyridine-2-carbonyl}amino}ethyl}carbamic acid 4-nitrobenzyl ester 31

Compounds **30** (0.39 g; 0.7 mmol) and **26** (0.43 g; 1.4 mmol) were dissolved in 20 ml dry acetonitrile. K₂CO₃ (0.48g; 3.5 mmol) was added and the mixture was refluxed for 3 hours. The precipitation was filtered off and solvent was evaporated. The product was purified on silica gel ( eluents: petroleum ether bp 40-60 °C: ethyl acetate: triethylamine 5:1:1 v/v/v ; diethylether ; 10% EtOH/CH₂Cl₂; 20% EtOH/ 10% TEA/CH₂Cl₂). ESI-TOF-MS mass for C₅₃H₅₆N₉O₁₂ (M+H)⁺: calcd, 1010.40; found 1010.46.

### Example 32

### The synthesis of 2-{{4-[6-(2-aminoethylaminocarbonyl)-4-(furan-2-yl)pyridin-2-methyl]-7-[2-carboxy-4-(furan-2-yl)pyridin-2-ylmethyl]-[1,4,7]triazacyclononanyl-1-methyl}-4-(furan-2-yl)pyridine-2-carboxylic acid 32

Compound **31** (0.80 g; 8.0 mmol) was dissolved in EtOH (30 ml). 10% Pd/C ( 0.49g) was added and the mixture was stirred under hydrogen atmosphere at RT for 2 hours. The mixture was filtered and solvent was evaporated. The residue was dissolved to 0.5M KOH/MeOH solution and the mixture was stirred at RT for 3 hours. Solvent was evaporated. The product was not purified before next step. ESI-TOF-MS mass for C₄₁H₄₂KN₈O₈ (M+K)⁺: calcd, 813.28; found 813.31.

### Example 33

### The synthesis of 2-{{4-[6-(2-aminoethylcarbamoyl)-4-(furan-2-yl)pyridin-2-methyl]-7-[2-carboxy-4-(furan-2-yl)pyridin-2-ylmethyl]-[1,4,7]triazacyclononanyl-1-methyl}-4-(furan-2-yl)pyridine-2-carboxylic acid lanthanide(III) 33

Compound 32 was converted to the corresponding europium(III) (**33a**) and samarium(III) (33b) chelates as described in Example 3.

### Example 34

### The synthesis of 2-{{4-[4-(furan-2-yl)-6-(2-iodoacetamidoethylcarboxamide)pyridin-2-methyl]-7-[2-carboxy-4-(furan-2-yl)pyridin-2-ylmethyl]-[1,4,7]triazacyclononanyl-1-methyl}-4-(furan-2-yl)pyridine-2-carboxylic acid lanthanide(III) 34.

Compounds 33 were converted to the corresponding iodoacetamido derivatives as descriibed in Takalo et al, Bioconjugate Chem., 1994, 5, 278.

### Example 35

Labeling of an oligopeptide on solid phase by using the labeling reactant 18.

The chain assembly, deprotection, introduction of the lanthanide(II) ion and purification was performed as described in Peuralahti et al., Bioconjugate Chem., 13, 2002, 870.

### Example 36

Labeling of an oligopeptide in solution using the chelate **34a**

The labeling reaction was performed using the method described in Takalo et al, Bioconjugate Chem., 1994, 5, 278.

### Example 37

Labeling of 8-ABA-cAMP with the chelate **23**.

8-ABA-cAMP (2.5 µmol) was dissolved in 500 µl of buffer containing pyridine, water and triethylamine (9.0 : 1.5 : 0.1 v/v/v). Compound **23** (3.0 µmoles; Ln = Eu) was added and the mixture was stirred at RT for 3 hours. The solvents were evaporated and the product was purified with HPLC. ESI-TOF MS: 1326.26.

### Example 38

### The synthesis of 1-(2-pyridyl)-3-(5-bromo-2-furyl)-E-propenone 35.

5-bromofuran-2-carboxaldehyde (5.0 g) was dissolved in a solution of KOH (1.5 g) in the mixture of water (10 mL) and methanol (50 mL) at 0 °C. 2-acetylpyridine (3.2 g) was added during 10 min, and the reaction was allowed to proceed for 3 h at room temperature. The product formed was isolated by filtration. Yield was 7.3 g.

### Example 39

### The synthesis of 4'-(5-bromo-2-furyl)-2,2':6',2"-terpyridine 36

Compound **35** (3.12 g, 11.3 mmol), *N*-[2-(pyrid-2'-yl)-2-oksoethyl]pyridinium jodide (3.69 g, 11.3 mmol) and ammonium acetate (21.8 g, 283 mmol) were dissolved in dry methanol (110 mL), and the mixture was heated overnight at reflux. The product formed was isolated by filtration after cooling to -18 °C. Yield was 2.5 g.

### Example 40

### The synthesis of 4'-(5-bromo-2-furyl)-2,2':6',2"-terpyridine N,N"dioxide 37

Synthesis was performed as described in Example 5. Yield was 78%. 1H NMR (CDC13): δ 8.38 (2H, dd, *J* 1.5 and 6.3); 8.22 (2H, dd, *J*2.2 and 6.3); 7.41 (2H, dt, *J* 1.5 and 7.8); 7.33 (2H, dt, *J* 2.2 and 6.3); 7.03 (1H, d, *J* 3.7); 6.49 (1H, d, *J* 3.7). ESI TOF MS for C₁₉H₁₂BrN₃O₃ (M+H)⁺: calcd, 410.01; found, 410.05.

### Example 41

### The synthesis of 4'-(5-bromo-2-furyl)-2,2':6',2 "-terpyridine-6,6'-dicarbonitrile 38

Synthesis was performed as described in Example 6. Compound 38: ¹H NMR (CDCl₃): δ 8.83 (2H, d, *J* 7.9); 8.72 (2H, s); 8.03 (2H, t, *J* 7.9); 7.80 (2H, d; *J* 7.9); 7.17 (1H, d, *J* 3.6); 6.57 (1H, d, *J* 3.6).

### Example 42

### The synthesis of 2,2'-2",2'''-{[4'-(5-bromo-2-furyl)-2,2':6',2"-terpyridine-6,6'-diyl]bis(methylenenitrilo)}tetrakis(acetate) tetra-tert-butyl ester 39

The synthesis was performed as described in example 7. Yield of compound 39 was 54%. ¹H NMR (CDCl₃): δ 8.66 (2H, s); 8.51 (2H, d, *J* 6.6); 7.86 (2H, t, *J* 7.7); 7.73 (2H, d, *J* 6.6); 7.09 (1H, d, *J* 3.6); 6.51 (1H, d, *J* 3.6); 4.20 (4H, s); 3.56 (8H, s); 1.49 (36H, s).

### Example 43

### The synthesis of 2,2'-2",2'''-{[4'-(5-(4-aminophenylethynyl-2-furyl)-2,2':6',2"-terpyridine-6,6'-diyl]bis(methylenenitrilo)}tetrakis(acetate) tetra-tert-butyl ester 40

Compound 39 (70 mg, 78 µmol) was dissolved in the mixture of dry THF (2 mL) and TEA (3 mL). Pd(Ph₃P)₂Cl₂ (1.1 mg) CuI (0.6 mg) were added and the mixture was deaerated with argon for 10 min. Aminophenylacetylene (11 mg, 94 µmol) was added and the mixture was heated overnight at 65 °C under argon atmosphere. All volatiles were removed in vacuo. The residue was dissolved in dichlromethane (3 mL), washed with water (3 · 2 mL) and dried (Na₂SO₄). Purification on silica gel (eluent PE:EA:TEA; 5:3:1; v/v/v) yielded 39 mg (53%) of compound 40. ¹H NMR (CDCl₃): δ 8.73 (2H, s); 8.52 (2H, d, *J* 7.5); 7.86 (2H, t, *J* 7.5); 7.73 (2H, d, *J* 7.5); 7.41 (2H, d, *J* 8.4); 7.14 (1H, d, *J* 3.6); 6.75 (1H, d, *J* 3.6); 6.68 (2H, d, *J* 8.4); 4.19 (4H, s); 3.56 (8H, s); 1.49 (36H, s). IR: 2270 cm⁻¹(-C≡C-).

### Example 44

### The synthesis of 2,2'-2",2'''-{[4'-(5-(4-aminophenylethyl-2-furyl)-2,2':6',2"-terpyridine-6,6'-diyl]bis(methylenenitrilo)}tetrakis(acetate) tetra-tert-butyl ester 41

Compound **40** (0.11 g, 0.12 mmol) was dissolved in dry methanol (50 mL). 10% Pd/C (20 mg) was added, and the mixture was stirred overnight at hydrogen atmosphere and filtered through Celite. Purification was performed on a preparative TLC plate (eluent petroleum ether : ethyl acetate: triethylamine; 5:3:1, v/v/v). ESI TOF MS for C₅₃H₆₉N₆O₉ (M+H)⁺: calcd, 933.6; found, 933.6

### Example 45

### The synthesis of 2,2'-2",2"'-{[4'-(5-(4-aminophenylethyl)-2-furyl)-2,2':6',2"-terpyridine-6,6'-diyl]bis(methylenenitrilo)}tetrakis(acetic acid) 42.

Compound **41** (14 mg, 15 µmol) was dissolved in TFA (1 mL) and stirred for 1 ½ h at room temperature before being concetrated, triturated with diethyl ether and filtered. Yield was 11 mg. ESI TOF MS for C₃₇H₃₆N₆O₉ (M-H)⁻: calcd, 707.3; found, 707.3.

### Example 46

### The synthesis of 2,2'-2",2'''-{[4'-(5-(4-aminophenylethyl)-2-furyl)-2,2':6',2"-terpyridine-6,6'-diyl]bis(methylenenitrilo)}tetrakis(acetic acid) europium(III) 43.

Compound **42** (8.5 mg, 9 µmol) was dissolved in water (400 µL) and the pH was adjusted to 6 with solid NaHCO₃. Europium(III) chloride (3.7 mg, 10 µmol; predissolved in 80 µL of water) was added portionwise (pH 5-7; adjusted with NaHCO₃) and the mixture was stirred at room temperature for 1½ h. pH was adjusted to 8.5 with 1 M NaOH, and the europium(III) hydroxide formed was removed by centrifugation. Precipitation with acetone yielded the title compound. ESI TOF MS for C₃₇H₃₂EuN₆O₉ (M-H)⁻: calcd, 857.14; found, 857.20.

### Example 47

### The synthesis of 2,2'-2",2"'-{[4'-(5-(4-isothiocyanatophenylethyl)-2-furyl)-2,2':6',2"-terpyridine-6,6'-diyl]bis(methylenenitrilo)}tetrakis(acetic acid) europium(III) 44.

Compound **43** (5.7 mg, 6.5 µmol; predissolved 170 µL of water 170) was added portionwise to the mixture of thiophosgene (2 µL), sat. NaHCO₃ (170 µL) and chloroform (170 µL). The mixture was strirred vigorously at room temperature for 1 ½h h, after which the phases were separated. The aqueous layer was washed with chloroform (2 · 170 µL) and precipitated from acetone to give compound **44**. ESI TOF MS for C₃₈H₃₀EuN₆O₉S⁻ (M-H)⁻: calcd, 899.1; found, 899.2.

### Example 48

### The synthesis of 6-(bromomethyl)-4-(5-bromofuran-2-yl)pyridine-2-carboxylic acid ethyl ester 45

Compound **26** (0.8 g, 2.58 mmol) was dissolved in dry dioxane (20 mL). Bromine (0.62 g, 3.87 mmol) was added, and the mixture was stirred overnight at room temperature. All volatiles were removed in vacuo. Purification on silica gel using dichlromethane as the eluent yielded the title compound.

### Example 49

### The synthesis of 7- {4-(5-bromofuran-2-yl)-6-[ethylcarbonyl]pyridin-2-ylmethyl}-[1,4,7]triazacyclononane-1,4-dicarboxylic acid di-tert-butyl ester 46

Compound 46 was synthesized using the method described in Example 29. ESI TOF MS for C₂₉H₄₂BrN₄O₇ (M+H)⁺: calcd, 637.2; found, 637.2

### Example 50

### The synthesis of 7-{4-(5-bromofuran-2-yl)-6-[ethoxycarbonyl]pyridin-2-ylmethyl}-[1,4,7]triazacyclononane 47

Synthesis was performed using the method described in Example 30. ESI TOF MS for C₁₉H₂₆BrN₄O₃ (M+H)⁺: calcd, 439.1; found, 439.1.

### Example 51

### The synthesis of 4,7-bis{6-[2-ethoxycarbonyl-4-(furan-2-yl)pyridin-2-yl]methyl}-1-{6-[2-ethoxycarbonyl]-4-(5-bromofuran-2-yl)pyrin-2-yl)methyl-[1,4,7-triazacyclononane 48

Reaction between compounds 46 and 26 using the method described in Example 31 followed by purification on basic aluminum oxide yielded the title compound. ESI TOF MS for C₄₅H₄₈BrN₆O₉ (M+H)⁺: calcd, 895.3; found, 895.4.

### Example 52

### The synthesis of 4,7-bis {6-[2-ethoxycarbonyl-4-(furan-2-yl)pyridin-2-yl]methyl}-1-{6-[2-ethoxycarbonyl]-4-[5-(6-(hydroxyhexyn-1-yl-)-furan-2-yl)pyrin-2-yl]methyl-[1,4,7-triazacyclononane 49

Reaction between compound **47** and hexynol using the method described in Example 43 yielded the title compound. Purification was performed on a column of neutral aluminum oxide. ESI TOF MS for C₅₁H₅₇N₆O₁₀(M+H)⁺: calcd, 913.4; found, 913.5.

### Example 53

### The synthesis of the oligonucleotide labeling reactant 50

Predried Compound **48** (0.114 g, 0.125 mmol) **1** and 2-cyanoethyl *N,N,N',N'-*tetraisopropylphosphordiamidite (1.5 eq) were dissolved in dry acetonitrile. 1*H* tetrazole (1 eq; 0.45 M in acetonitrile) was added, and the mixture was stirred for 30 min at room temperature before being poured into 5% NaHCO₃ and extracted with dichloromethane and dried over Na₂SO₄. Purification on basic aluminum oxide yielded the title compound: ³¹P NMR (CDCl₃): δ 151.2.

### Example 54

Introduction of a lanthanide(III) chelate to the oligonucleotide structure using compound **50** was performed using methods described in Hovinen and Hakala, Org. Lett. 3, 2001, 2473.

The oligonucleotide d(AAT CAG ACT GTT CAA GAC) was synthesized in conventional manner, and the reactant **50** was coupled to its 5'-terminus. Deprotection, convertion to the corresponding lanthanide(III) chelate and purification was performed as described in Org. Lett. 3, 2001, 2473.

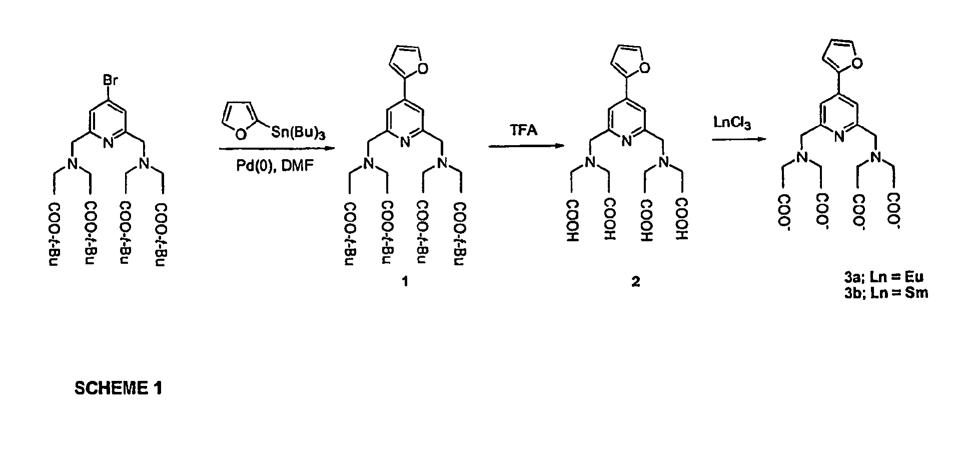

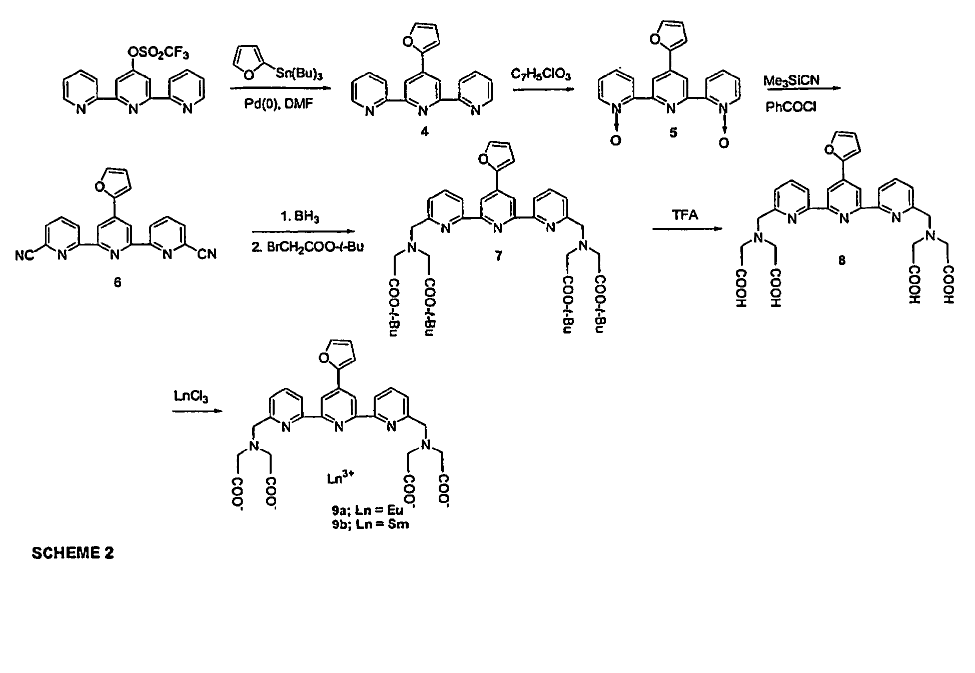

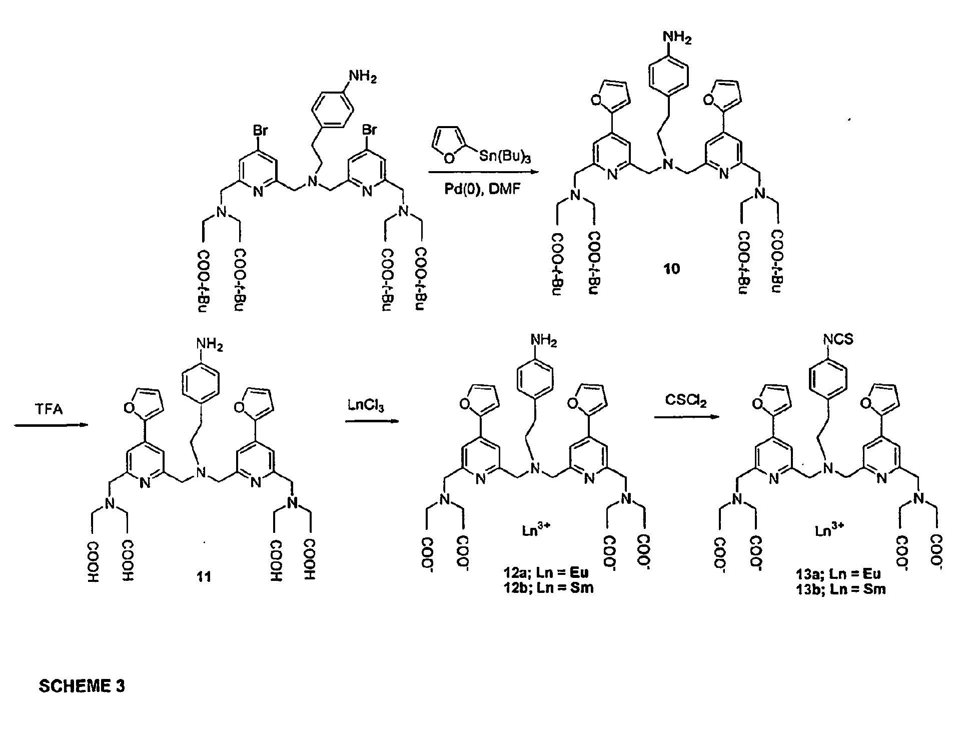

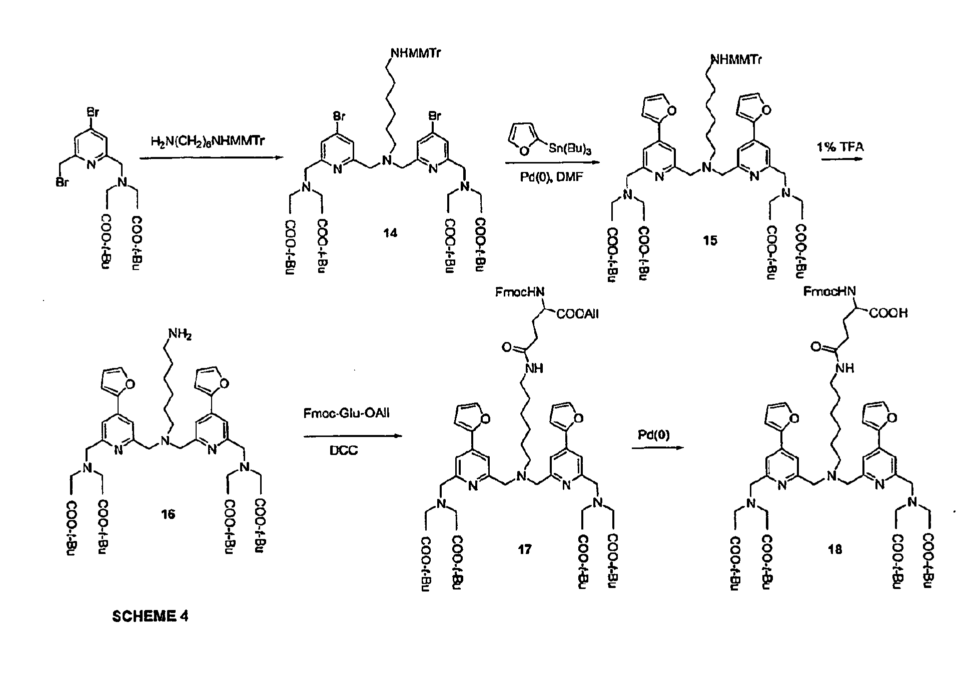

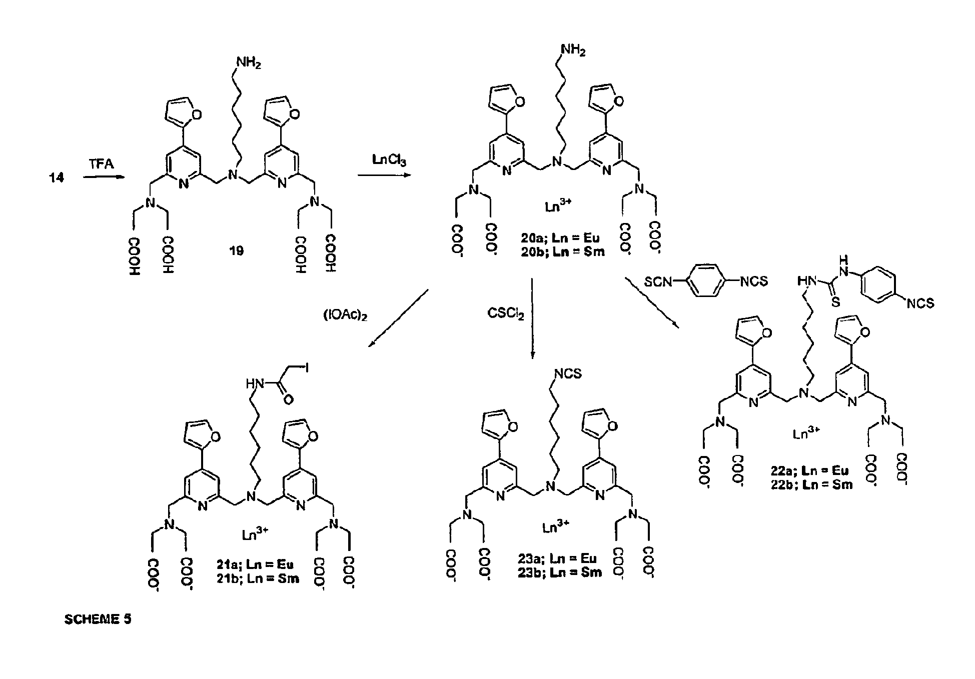

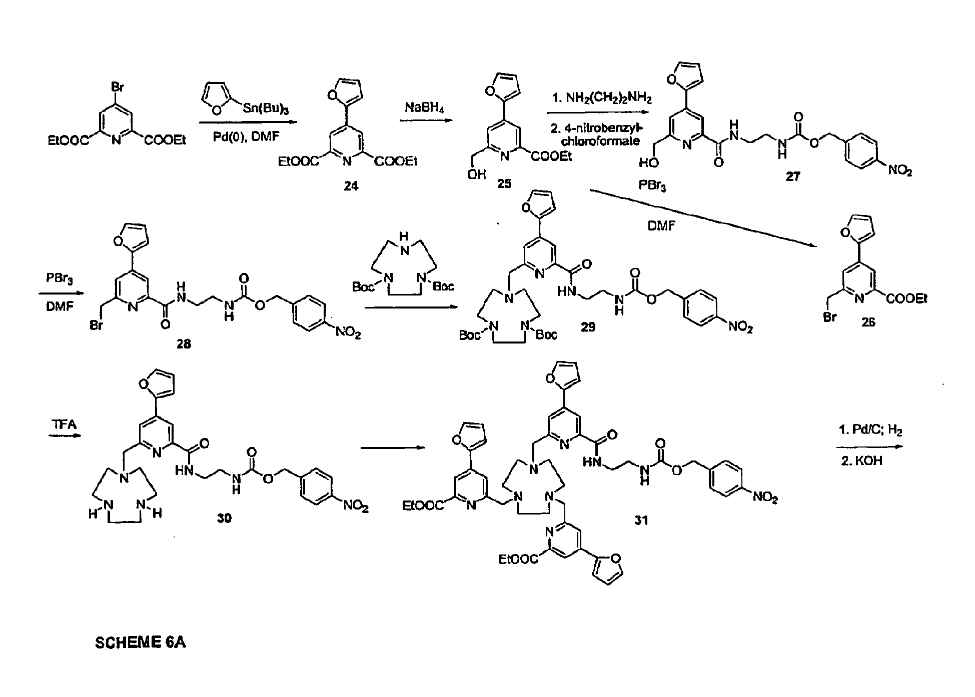

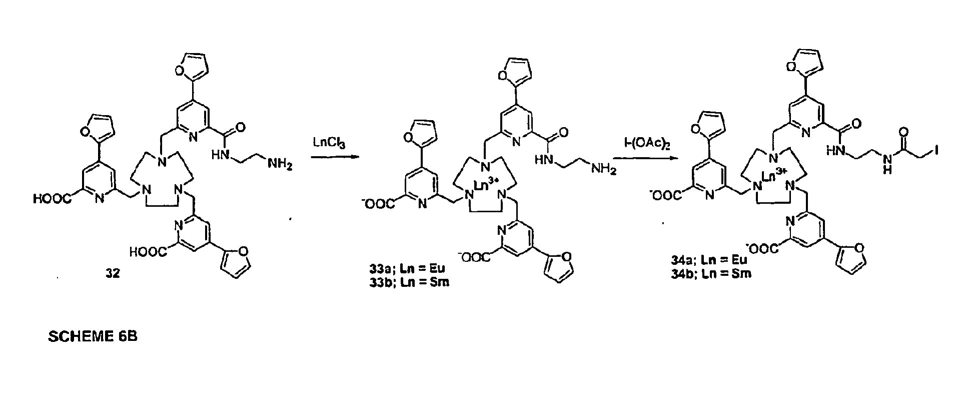

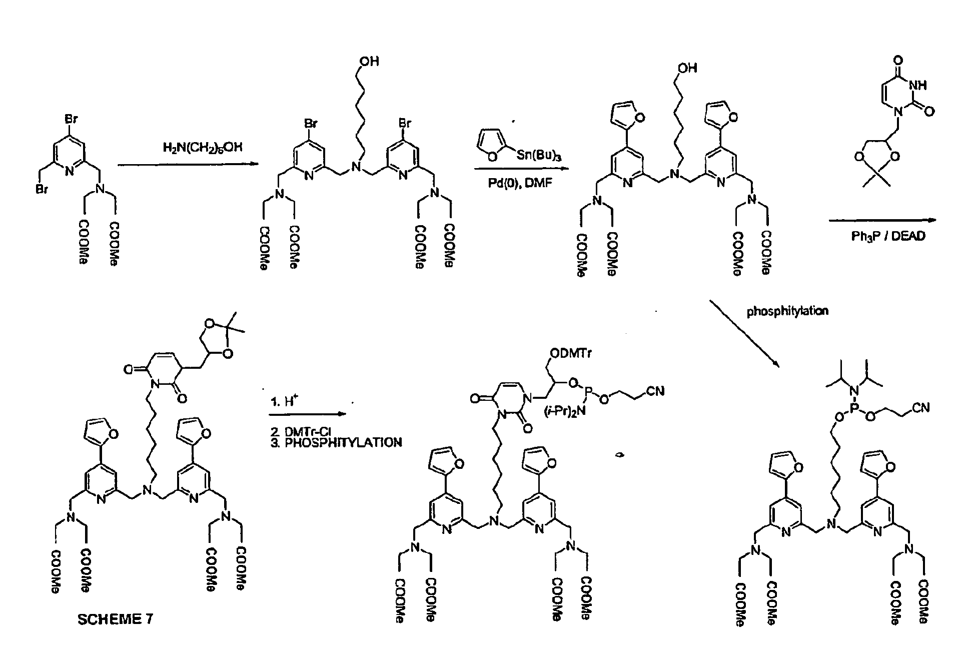

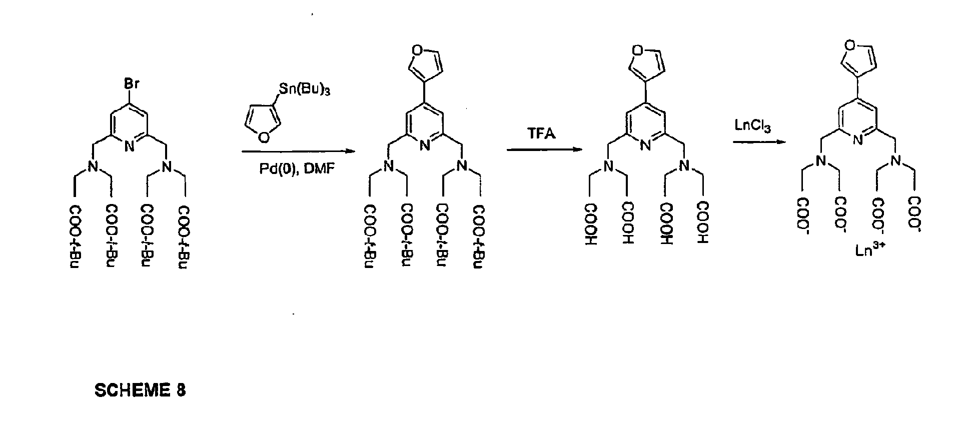

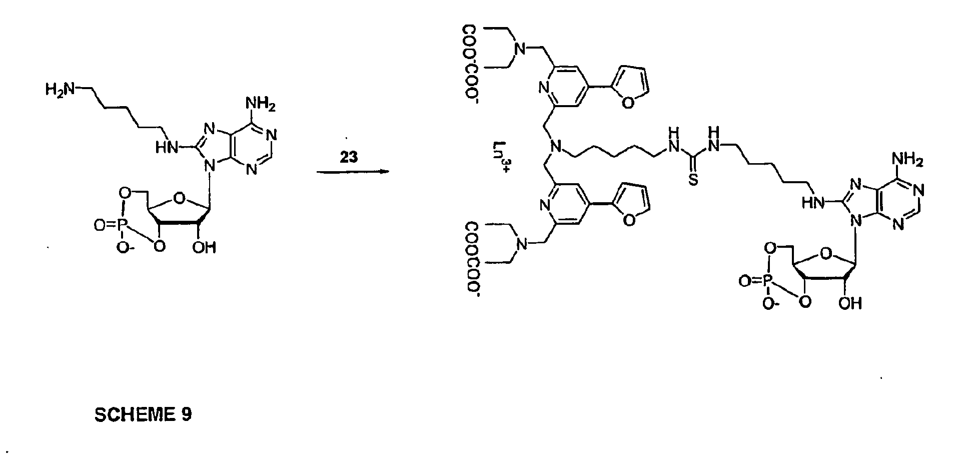

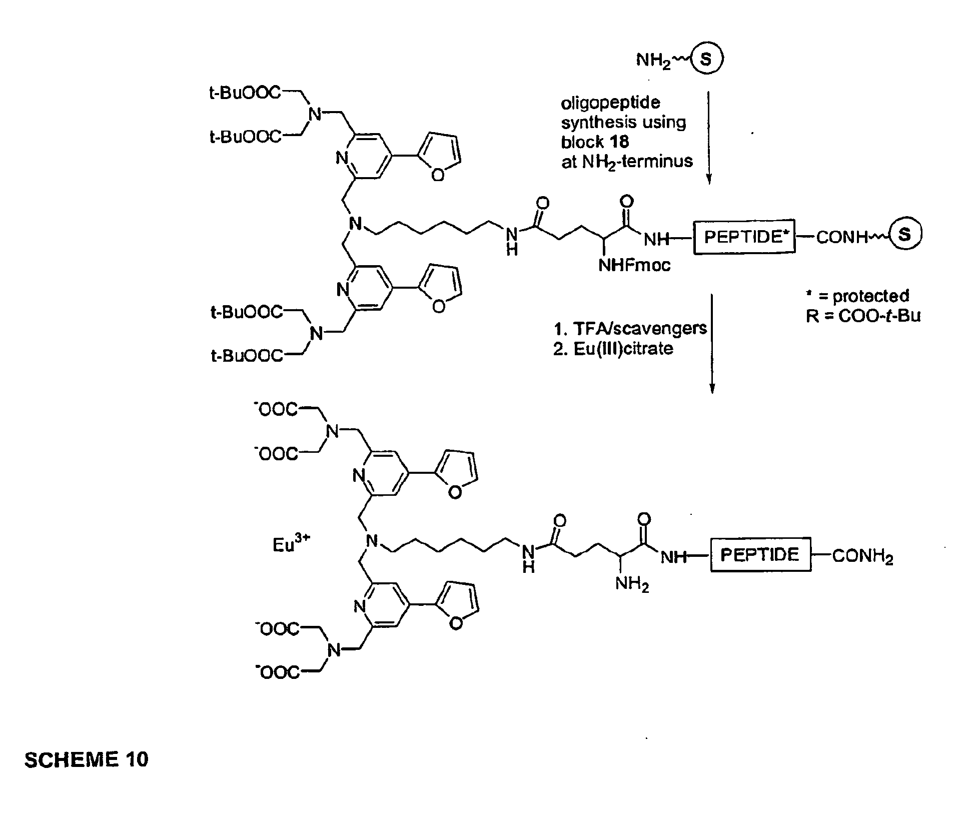

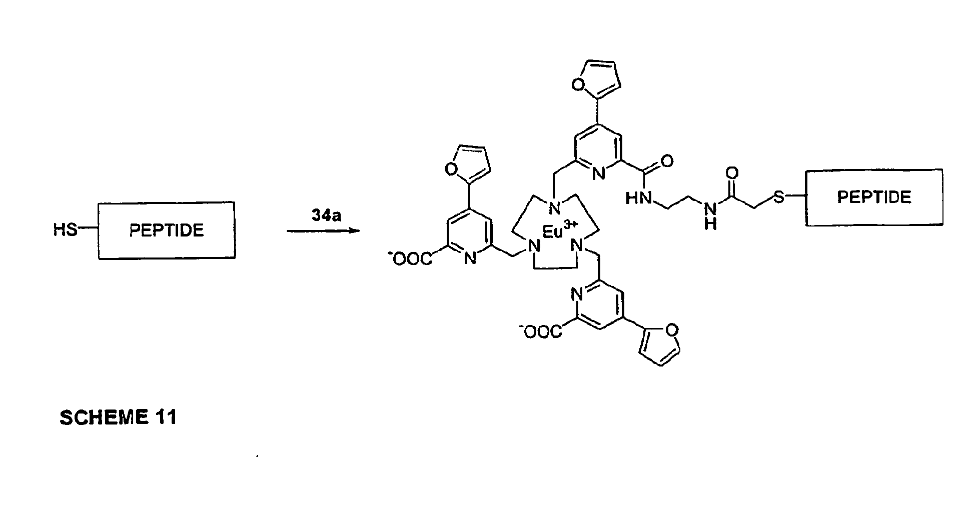

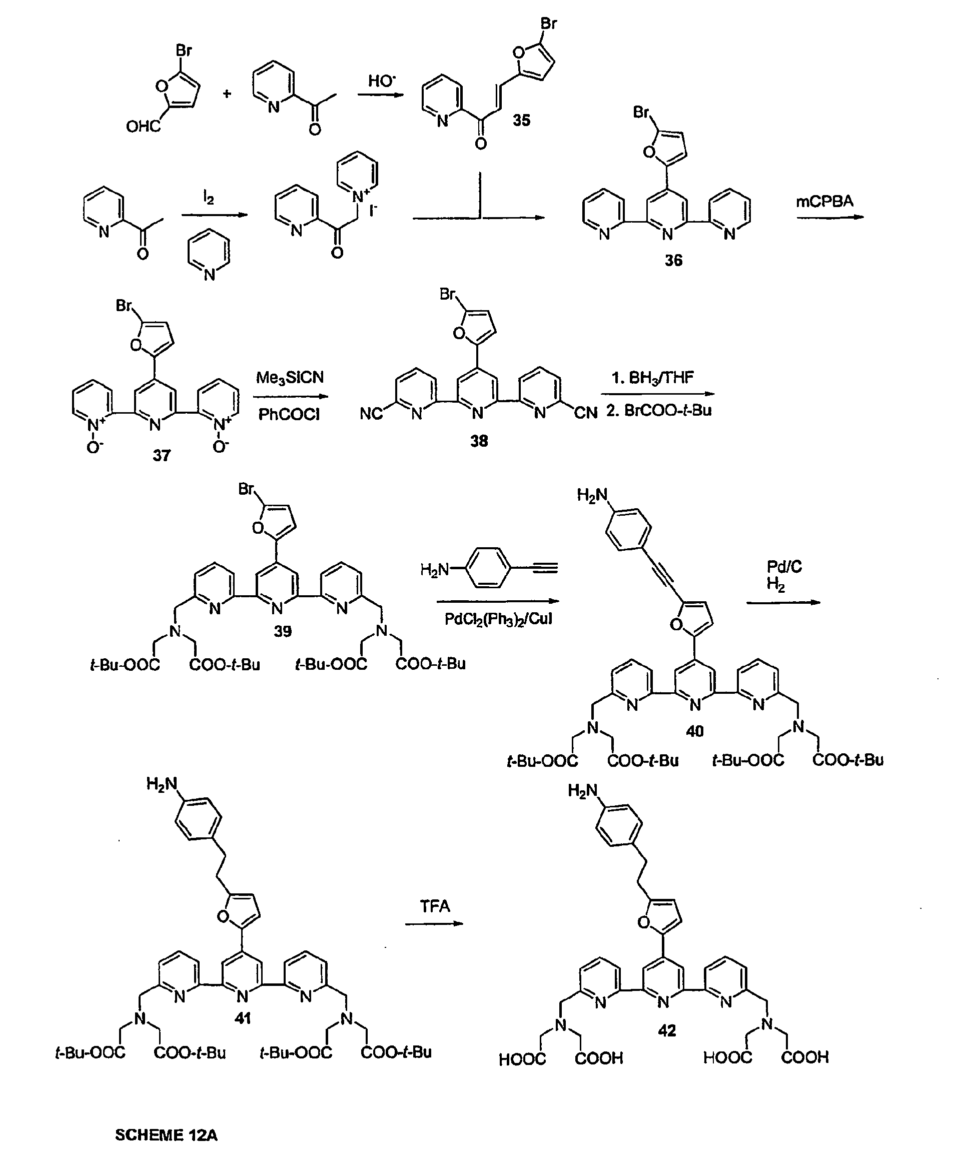

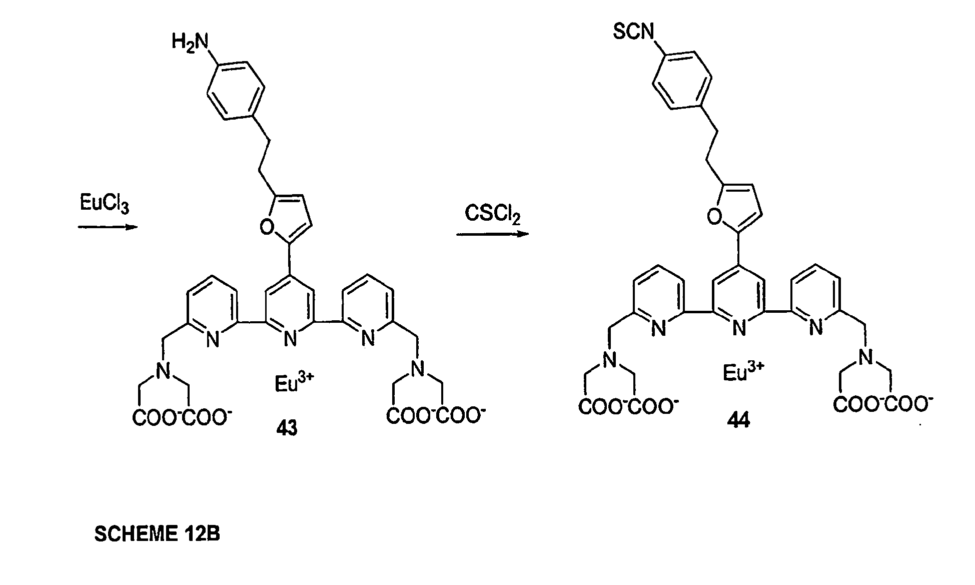

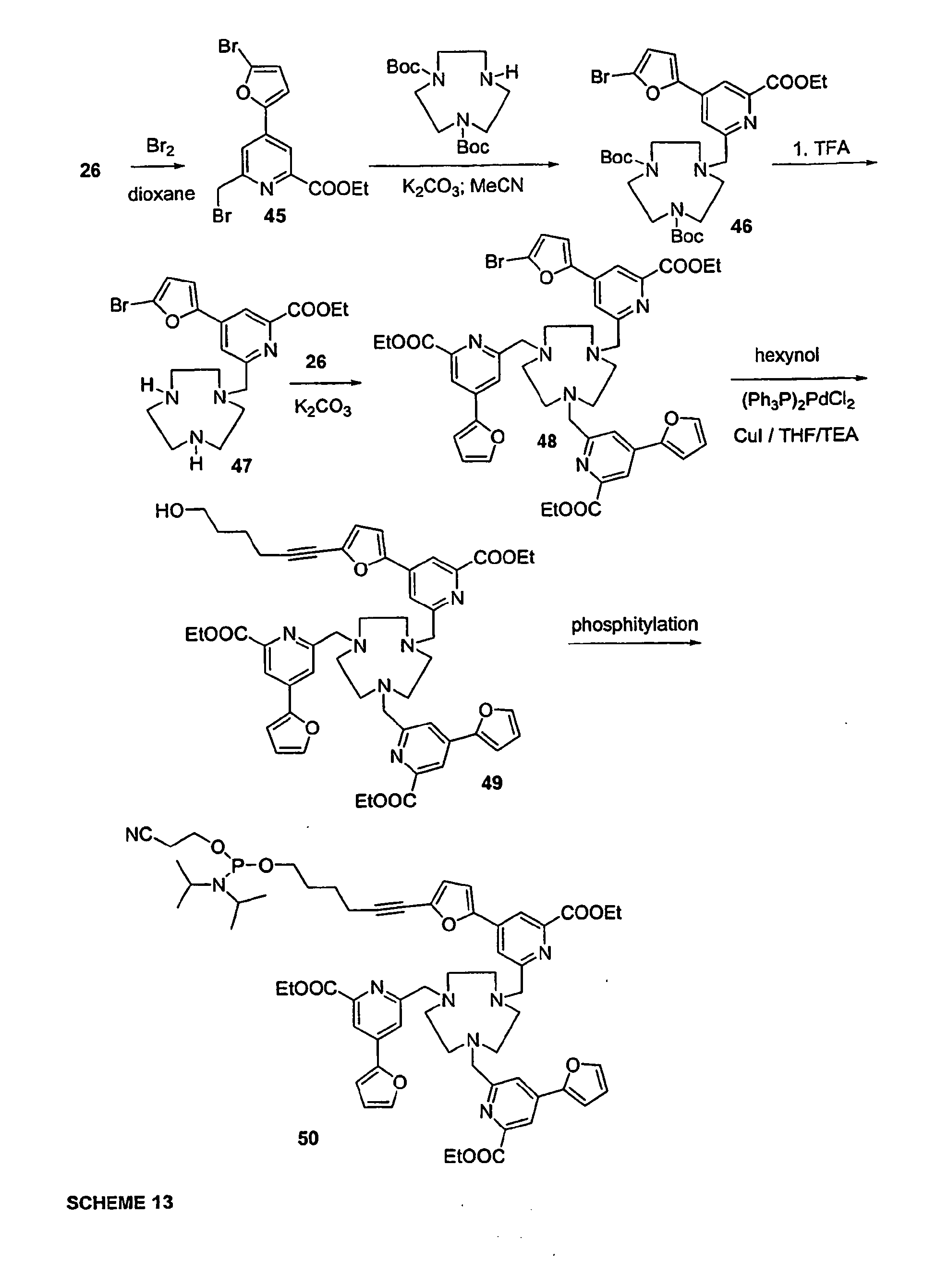

Photochemical properties of certain chelates according to this invention are shown in Table 1.

The photochemical properties of the chelates and peptide-coupled chelates were determined by measuring excitation and emission spectra and fluorescence lifetime in TS buffer (50 mM tris, 150 mM NaCl, pH 7.75) with LS-55 luminescence spectrometer (PerkinEhner Instuments, Connecticut, USA). Measurements were done with appropriate concentrations depending on expected fluorescence intensity.

**Table 1. Photochemical properties of the chelates synthesized**

| Compound | Excitation wavelenght / nm | Excitation wavelenght / nm | Lifetime / ms | Luminescence yield (∈Φ) |
|---|---|---|---|---|
| **3a** | 316 | 615 | 0.41 | 523 |
| **3b** | 326 | 598 | 0.008 | 2 |
| **9a** | 340 | 615 | 1.08 | 2100 |
| **9b** | 348 | 605 | 0.014 | 12 |
| **12a** | 318 | 616 | 0.96 | 470 |
| **13a** | 314 | 615 | 1.07 | 1100 |
| **20a** | 313 | 615 | 1.11 | 2700 |
| **20b** | 321 | 598 | 0.019 | 13 |
| **21b** | 306 | 644 | 0.0193 | 114 |
| **21b** coupled to a peptide | 313 | 645 | 0.0918 | 167 |
| **22a** | 316 | 615 | 0.98 | 610 |
| **33a** | 328 | 618 | 0.80 | t.d |
| **33b** | 333 | 645 | 0.0161 | t.d. |

| | | | | |
|---|---|---|---|---|
| t.d. = to be determined | | | | |

It will be appreciated that the methods of the present invention can be incorporated in the form of a variety of embodiments, only a few of which are disclosed herein. Thus, the described embodiments are illustrative and should not be construed as restrictive.

## Claims

1. A chelate comprising
- a lanthanide ion,
- a chromophoric moiety,
- a chelating part comprising at least two carboxylic acid or phosphonic acid groups, or esters or salts of said acids, attached to an aromatic unit of the chromophoric moiety, either directly or via an N-containing hydrocarbon chain, and
- a reactive group A, tethered to the chromophoric moiety or to the chelating part via a linker x, wherein
(i) the chromophoric moiety comprises two or three pyridyl groups, wherein at least one of them is furylsubstituted and said pyridyl groups either being tethered directly to each other to form terpyridyl group or to each other via N-containing hydrocarbon chains,
(ii) the linker x is formed from one to ten moieties, each moiety being selected from the group consisting of phenylene, alkylene containing 1-12 carbon atoms, ethynyldiyl (-C≡C-), ethylenediyl (-C=C-), ether (-O-), thioether (-S-), amide (-CO-NH-, -CO-NR'-, NH-CO and -NR'-CO-), carbonyl (-CO-), ester (-COO- and -OOC-), disulfide (-SS-), diaza (-N=N-), and tertiary amine, wherein R' represents an alkyl group containing less than 5 carbon atoms,
(iii) the reactive group A is selected from the group consisting of isothiocyanate, haloacetamido, maleimido, dichlorotriazinyl, dichlorotriazinylamino, pyridyldithio, thioester, aminooxy, hydrazide, amino, a polymerizing group, and a carboxylic acid or acid halide or an active ester thereof.

2. The chelate according to claim 1, selected from the group consisting of 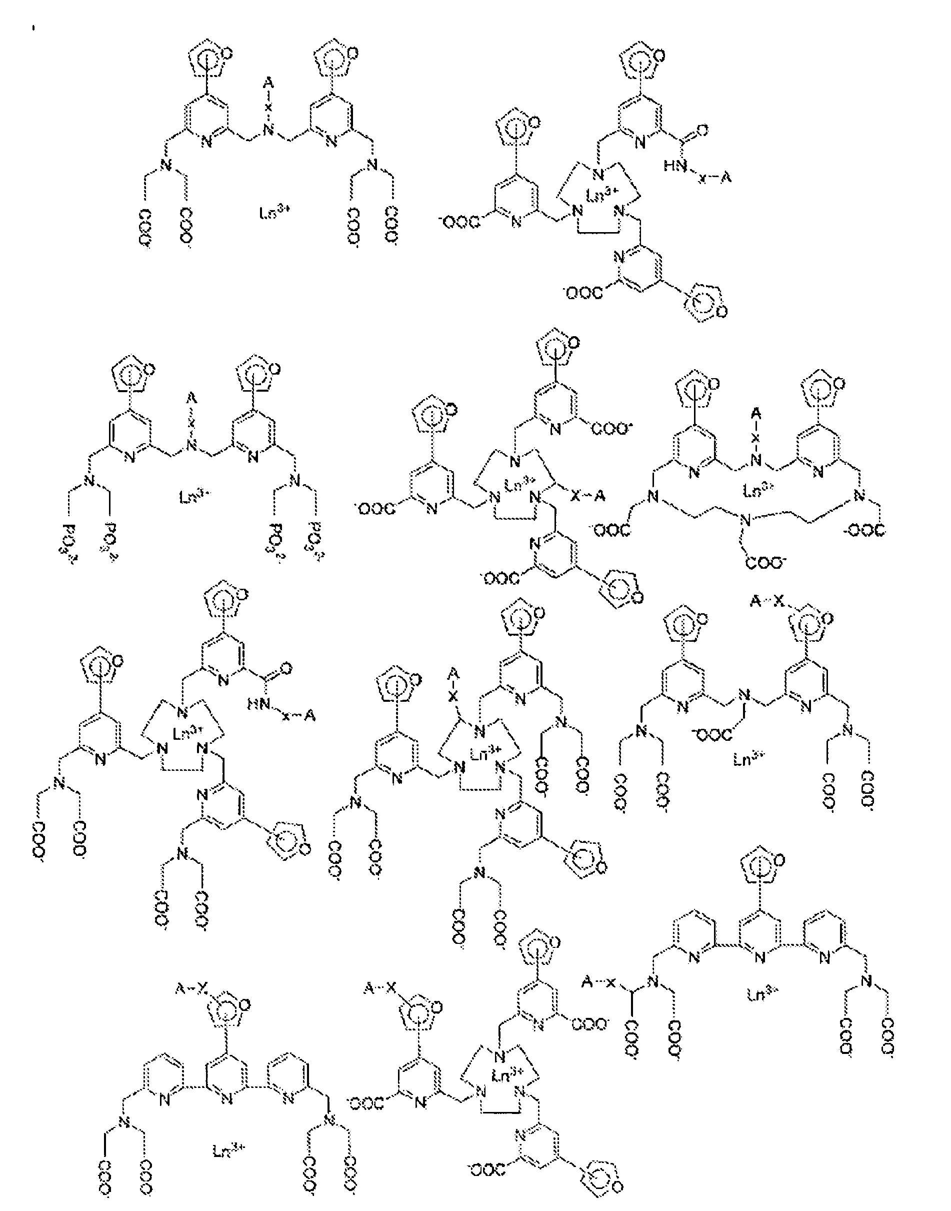 wherein x and A are as defined in claim 1.

3. The chelate according to claim 1, selected from the group consisting of 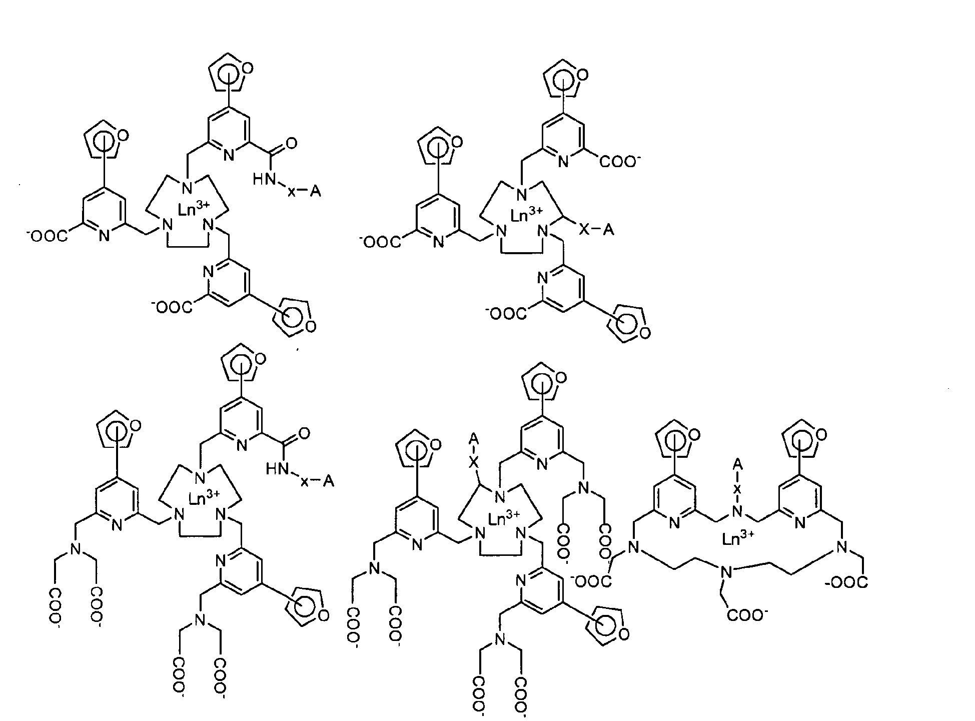 wherein x and A are as defined in claim 1.

4. The chelate according to claims 1-3 wherein the lanthanide ion is europium, samarium, terbium or dysprosium ion.

5. A chelating agent comprising
- a chromophoric moiety,
- a chelating part comprising at least two carboxylic acid or phosphonic acid ester groups, attached to an aromatic unit of the chromophoric moiety, either directly or via an N-containing hydrocarbon chain, and
- a reactive group A, tethered to the chromophoric moiety or to the chelating part via a linker x, wherein
(i) the chromophoric moiety comprises two or three pyridyl groups, wherein at least one of them is furylsubstituted and said pyridyl groups being tethered either directly to each other to form terpyridyl group or to each other via N-containing hydrocarbon chains,
(ii) the linker x is formed from one to ten moieties, each moiety being selected from the group consisting of phenylene, alkylene containing 1-12 carbon atoms, ethynydiyl (-C≡C-), ethylenediyl (-C=C-)ether (-O-), thioether (-S-), amide (-CO-NH-, -CO-NR'-NH-CO and -NR'-CO-), carbonyl (-CO-), ester (-COO- and -OOC-), disulfide (-SS-), diaza (-N=N-), and tertiary amine, wherein R' represents an alkyl group containing less than 5 carbon atoms,
(iii) A is an amino acid residue -CH(NHR₁)R₅ where R₁ is a transient protecting group and R₅ is a carboxylic acid or its salt, acid halide or an ester.

6. The chelating agent according to claim 5, selected from a group consisting of 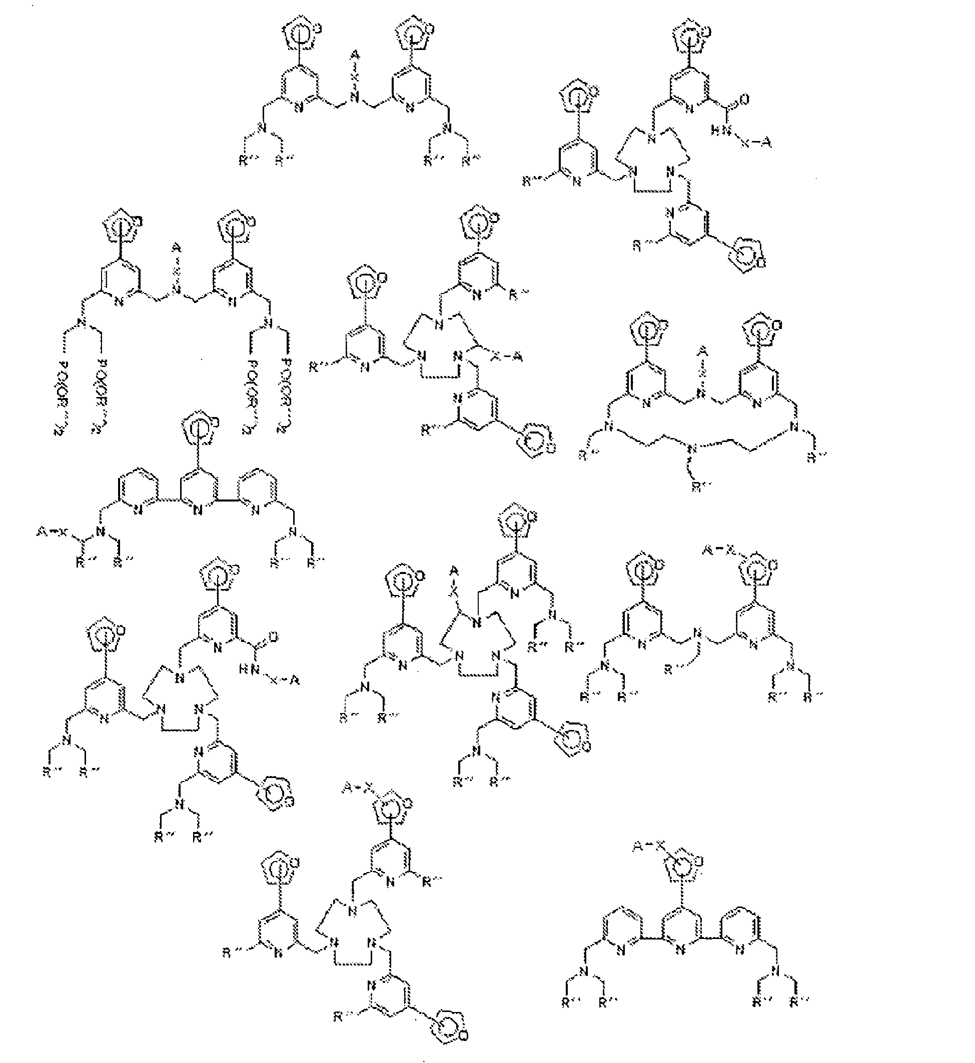 wherein x is as defined in claim 5 and the protecting group R₁ is selected from a group consisting of Fmoc, Boc, or Bsmoc, and R" is an alkyl ester or an allyl ester and R'" is an alkyl group.

7. A chelating agent comprising
- a chromophoric moiety,
- a chelating part comprising at least two carboxylic acid or phosphonic acid ester groups, attached to an aromatic unit of the chromophoric moiety, either directly or via an N-containing hydrocarbon chain,
- a reactive group A, tethered to the chromophoric moiety or to the chelating part via a linker x, wherein
(i) the chromophoric moiety comprises two or three pyridyl groups, wherein at least one of them is furylsubstituted and the said pyridyl groups being tethered either directly to each other to form terpyridyl group or to each other via N-containing hydrocarbon chains
(ii) the reactive group A is
-Y-O-PZ-O-R₄
where
one of the oxygen atoms optionally is replaced by sulfur,
Z is chloro or NR₂R₃
R₄ is a protecting group,
R₂ and R₃ are alkyl groups,
Y is absent or is a radical of a purine base or a pyrimidine base or any other modified base suitable for use in the synthesis of modified oligonucleotides, said base being connected to the oxygen atom via either
a) a hydrocarbon chain, which is substituted with a protected hydroxyethyl group, or via
b) a furan ring or pyrane ring or any modified furan or pyrane ring, suitable for use in the synthesis of modified oligonucleotides,
(iii) the linker x is formed from one to ten moieties, each moiety being selected from the group consisting of phenylene, alkylene containing 1-12 carbon atoms, ethynydiyl (-C≡C-), ethylenediyl (-C=C-)ether (-O-), thioether (-S-), amide (-CO-NH-, -CO-NR'-, NH-CO and -NR'-CO-), carbonyl (-CO-), ester (-COO- and -OOC-), disulfide (-SS-), diaza (-N=N-), and tertiary amine, wherein R' represents an alkyl group containing less than 5 carbon atoms,

8. The chelating agent according to claim 7 wherein Y is a radical of any of the bases thymine, uracil, adenosine, guanine or cytosine, and said base is connected to the oxygen atom via
i) a hydrocarbon chain, which is substituted with a protected hydroxyethyl group, or
ii) a furan ring having a protected hydroxyethyl group in its 4-position and optionally a hydroxyl, protected hydroxyl or modified hydroxyl group in its 2-position.

9. The chelating agent according to claim 8, wherein -Y-O-P(NR₂R₃)-O-R₄ is selected from the group consisting of 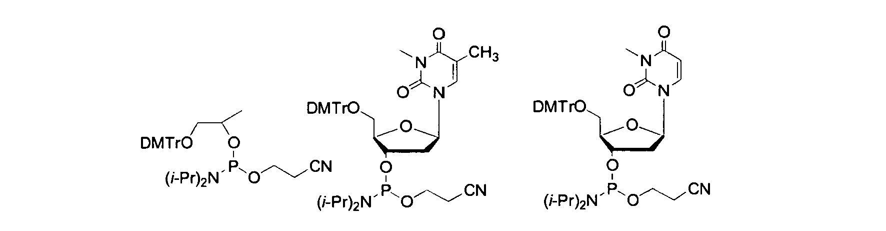 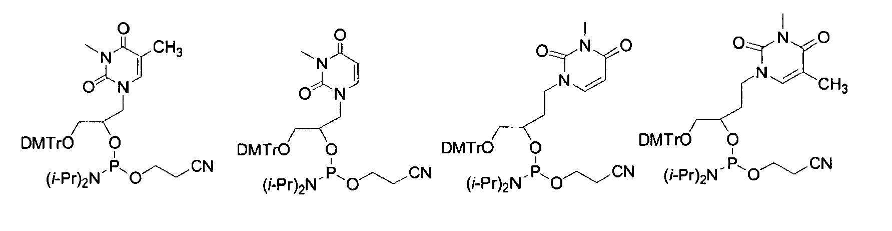 wherein "-" is the position of linker x and DMTr is dimethoxytrityl.

10. The chelating agent according to claim 9, selected from the group consisting of 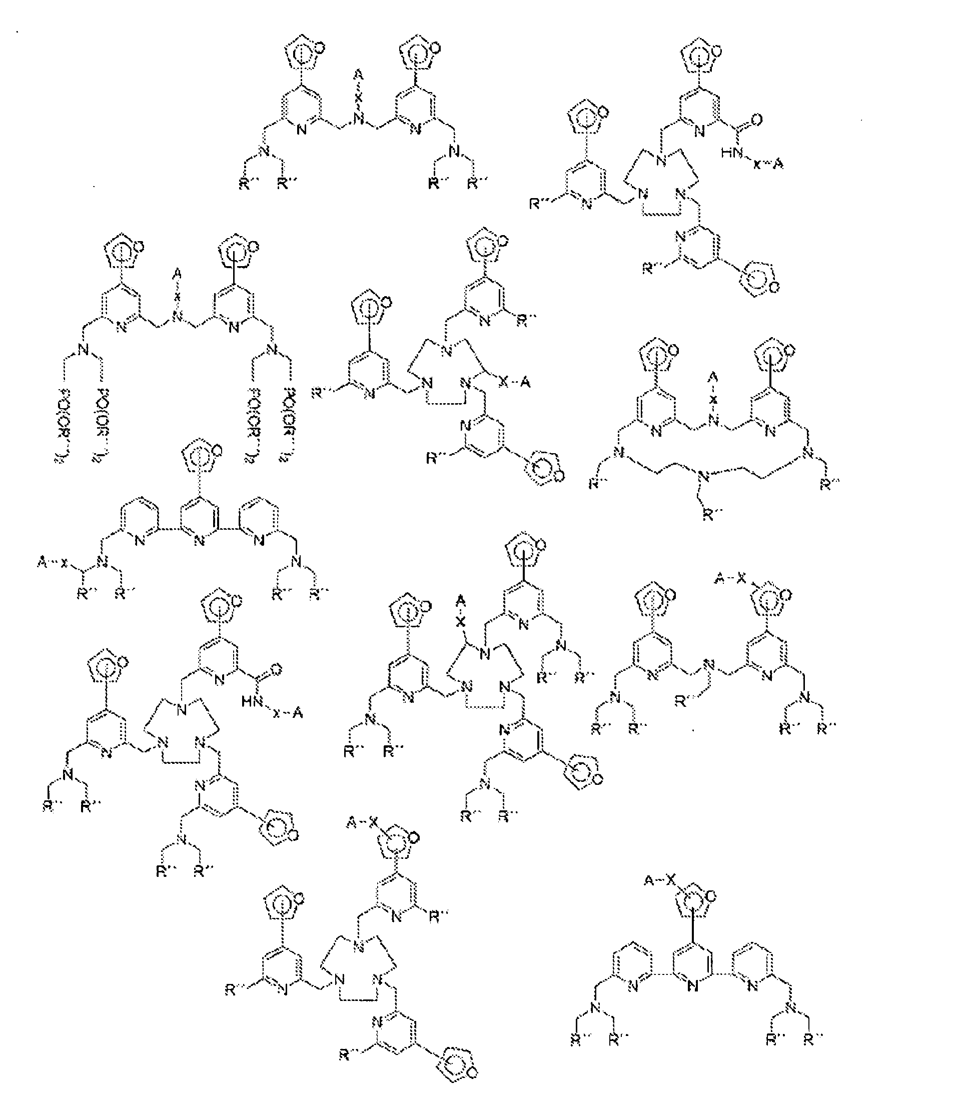 wherein R" is an alkyl ester or an allyl ester and R"' is an alkyl group and x is as defined in claim land A is -Y-O-P(NR₂R₃)-O-R₄ as defined in claim 7.

11. A biomolecule conjugated with a chelate according to any of the claims 1-4.

12. A biomolecule conjugated with a chelate or a chelating agent according to any of the claims 1-10 wherein the biomolecule is selected from the group consisting of an oligopeptide, oligonucleotide, DNA, RNA, modified oligo- or polynucleotide, protein, oligosaccaride, polysaccaride, phospholipide, PNA, LNA, antibody, hapten, drug, receptor binding ligand and lectine.

13. The biomolecule according to claim 12 wherein the modified oligo- or polynucleotide is a phosphoromonothioate, phosphorodithioate, phosphoroamidate and/or sugar- or basemodified oligo- or polynucleotide.

14. A biomolecule conjugated with a chelating agent according to any of the claims 5-10

15. A solid support conjugated with a chelate according to any of the claims 1-4.

16. A solid support conjugated with a chelate according to any of the claims 1-4, wherein said solid support is selected from the group consisting of a nanoparticle, a microparticle, a slide or a plate.

17. A labeled chelate coupled oligopeptide, obtained by synthesis on a solid phase, by introduction of a chelating agent according to claim 5 or 6 into the oligopeptide structure on an oligopeptide synthesizer, followed by deprotection and optionally also introduction of a metal ion.

18. A labeled chelate coupled oligonucleotide, obtained by synthesis on a solid phase, by introduction of a chelating agent according to any of the claims 7-10 into the oligonucleotide structure on an oligonucleotide synthesizer, followed by deprotection and optionally also introduction of a metal ion.

19. A solid support conjugated with a labeled oligopeptide according to claim 17 or a labeled oligonucleotide according to claim 18, wherein said oligopeptide or oligonucleotide is covalently or noncovalently immobilized on said solid support.

20. A solid support conjugated with a labeled oligopeptide according to claim 17 or a labeled oligonucleotide according to claim 18, wherein said oligopeptide or oligonucleotide is covalently or noncovalently immobilized on said solid support, which is selected from the group consisting of a nanoparticle, a microparticle, a slide or a plate.

21. A solid support conjugated with the chelating agent according to claim 5, suitable for use in the synthesis of an oligonucleotide, wherein the reactive group A is
-Y-O-x'-
where
x' is a linker connected to the solid support, and can be the same or different as the linker x
Y is absent or is a radical of a purine or pyrimidine or any other modified base suitable for use in the synthesis of modified oligonucleotides, said base being connected to the oxygen atom via either
i) a hydrocarbon chain, which is substituted with a protected hydroxyethyl group, or
ii) a furan ring or pyrane ring or any modified furan or pyrane ring, suitable for use in the synthesis of modified oligonucleotides.

## Patentansprüche

1. Ein Chelat umfassend
- ein Lanthanid-Ion,
- eine chromophore Gruppe,
- einen chelatbildenden Teil umfassend mindestens zwei Carbonsäure- oder Phosphonsäuregruppen, oder Ester oder Salze dieser Säuren, welche entweder direkt oder über eine N-haltige Kohlenwasserstoffkette an die aromatische Einheit der chromophoren Gruppe gebunden sind, und
- eine reaktive Gruppe A, gebunden an die chromophore Gruppe oder den chelatbildenden Teil über einen Linker x, wobei
(i) die chromophore Gruppe zwei oder drei Pyridyl-Gruppen umfasst, wobei zumindest eine von diesen furylsubstituiert ist, und besagte Pyridylgruppen entweder direkt aneinander angebunden sind und so eine Terpyridyl-Gruppe bilden, oder über N-haltige Kohlenwasserstoffketten aneinander gebunden sind,
(ii) der Linker x aus einer bis zehn Einheiten zusammengesetzt ist, wobei jede Einheit ausgewählt ist aus der Gruppe bestehend aus Phenylen, Alkylen mit 1-12 Kohlenstoffatomen, Ethinyldiyl (-C≡C-), Ethylendiyl (-C=C-), Ether (-O-), Thioether (-S-), Amid (-CO-NH-, - CO-NR'-, -NH-CO- und -NR'-CO-), Carbonyl (-CO-), Ester (-COO- und -OOC-), Disulfid (-SS-), Diaza (-N=N-), und tertiärem Amin, worin R' eine Alkylgruppe mit weniger als 5 Kohlenstoffatomen bedeutet,
(iii) die reaktive Gruppe A ausgewählt ist aus der Gruppe bestehend aus Isothiocyanat, Halogenacetamido, Maleimido, Dichlortriazinyl, Dichlortriazinylamino, Pyridyldithio, Thioester, Aminooxy, Hydrazid, Amino, einer polymerisierenden Gruppe, und einer Carbonsäure oder -säurehalogenids oder eines aktiven Esters hiervon.

2. Das Chelat gemäß Anspruch 1, ausgewählt aus der Gruppe bestehend aus 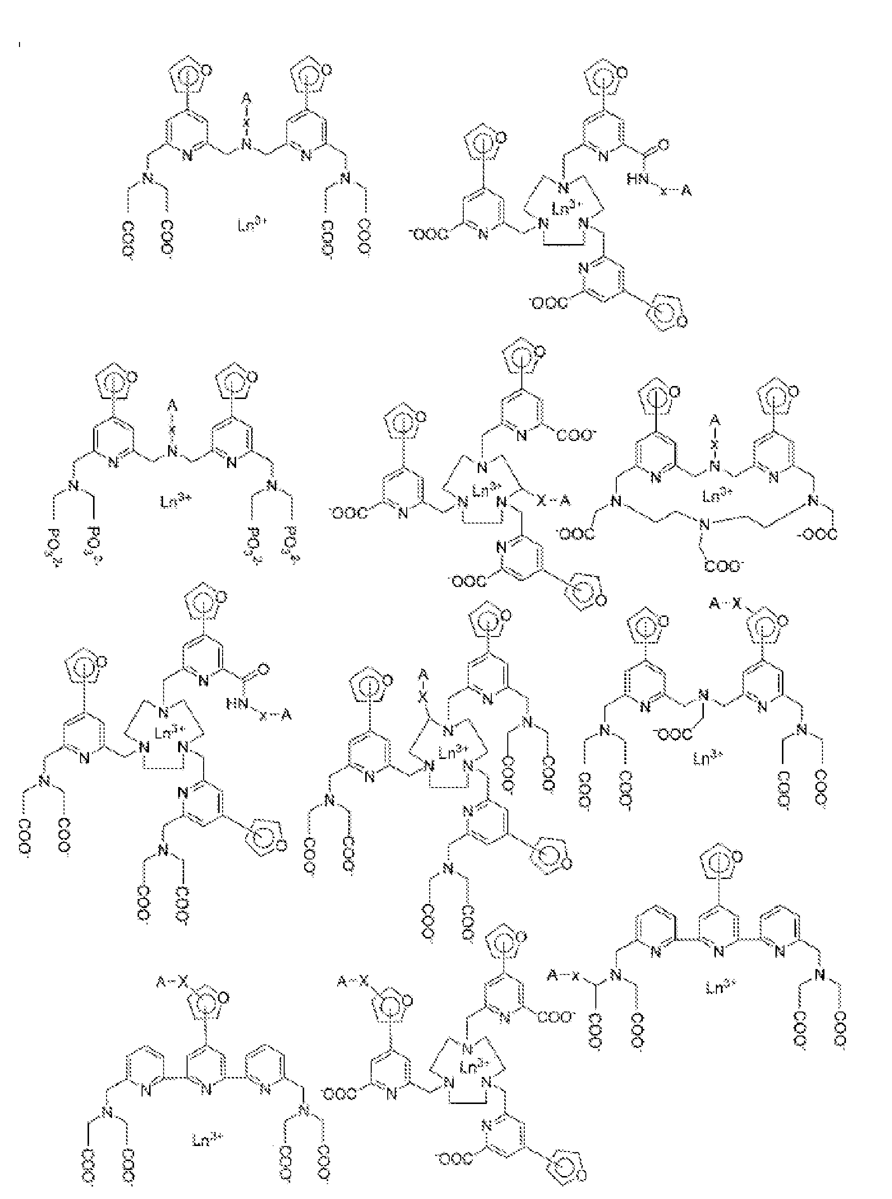 worin x und A wie in Anspruch 1 definiert sind.

3. Das Chelat gemäß Anspruch 1, ausgewählt aus der Gruppe bestehend aus 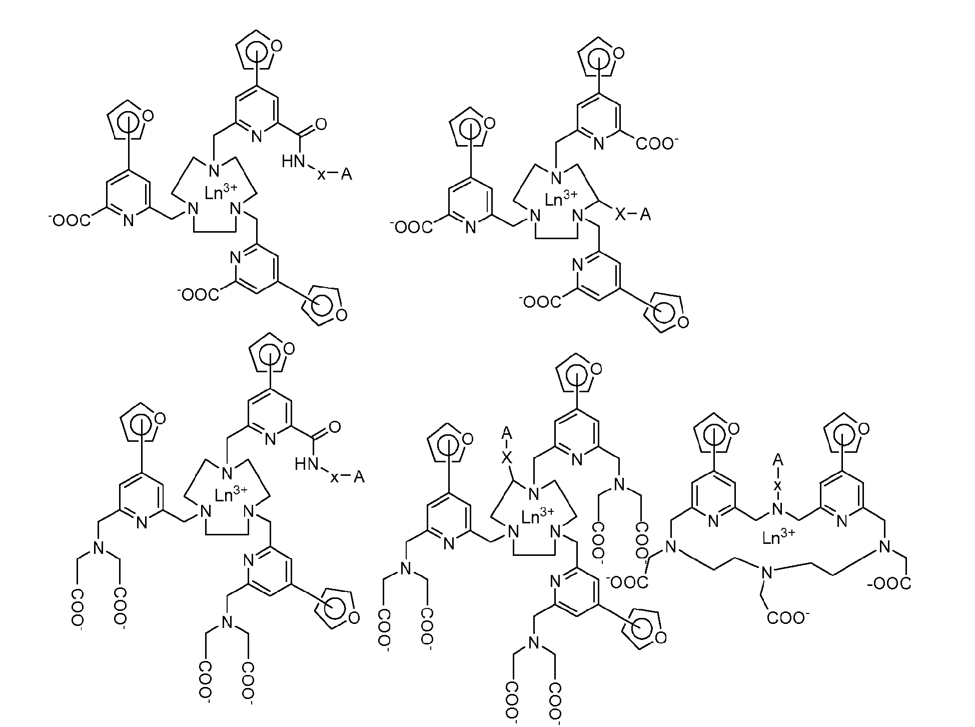 worin x und A wie in Anspruch 1 definiert sind.

4. Das Chelat gemäß der Ansprüche 1-3, worin das Lanthanid-Ion ein Europium-, Samarium-, Terbium -oder Dysprosium-Ion ist.

5. Ein Chelatbildner umfassend
- eine chromophore Gruppe,
- einen chelatbildenden Teil umfassend mindestens zwei Carbonsäure- oder Phosphonsäureestergruppen, welche entweder direkt oder über eine N-haltige Kohlenwasserstoffkette an die aromatische Einheit der chromophoren Gruppe gebunden sind, und
- eine reaktive Gruppe A, gebunden an die chromophore Gruppe oder den chelatbildenden Teil über einen Linker x, wobei
(i) die chromophore Gruppe zwei oder drei Pyridyl-Gruppen umfasst, wobei zumindest eine von diesen furylsubstituiert ist, und besagte Pyridylgruppen entweder direkt aneinander gebunden sind und so eine Terpyridyl-Gruppe bilden, oder über N-haltige Kohlenwasserstoffketten aneinander gebunden sind,
(ii) der Linker x aus einer bis zehn Einheiten zusammengesetzt ist, wobei jede Einheit ausgewählt ist aus der Gruppe bestehend aus Phenylen, Alkylen mit 1-12 Kohlenstoffatomen, Ethinyldiyl (-C≡C-), Ethylendiyl (-C=C-), Ether (-O-), Thioether (-S-), Amid (-CO-NH-, - CO-NR'-, -NH-CO- und -NR'-CO-), Carbonyl (-CO-), Ester (-COO- und -OOC-), Disulfid (-SS-), Diaza (-N=N-), und tertiärem Amin, worin R' eine Alkylgruppe mit weniger als 5 Kohlenstoffatomen bedeutet,
(iii) A ein Aminosäurerest -CH(NHR₁)R₅ ist, worin R₁ eine transiente Schutzgruppe und R₅ eine Carbonsäure oder deren Salz, Säurehalogenid oder Ester ist.

6. Der Chelatbildner gemäß Anspruch 5, ausgewählt aus der Gruppe bestehend aus 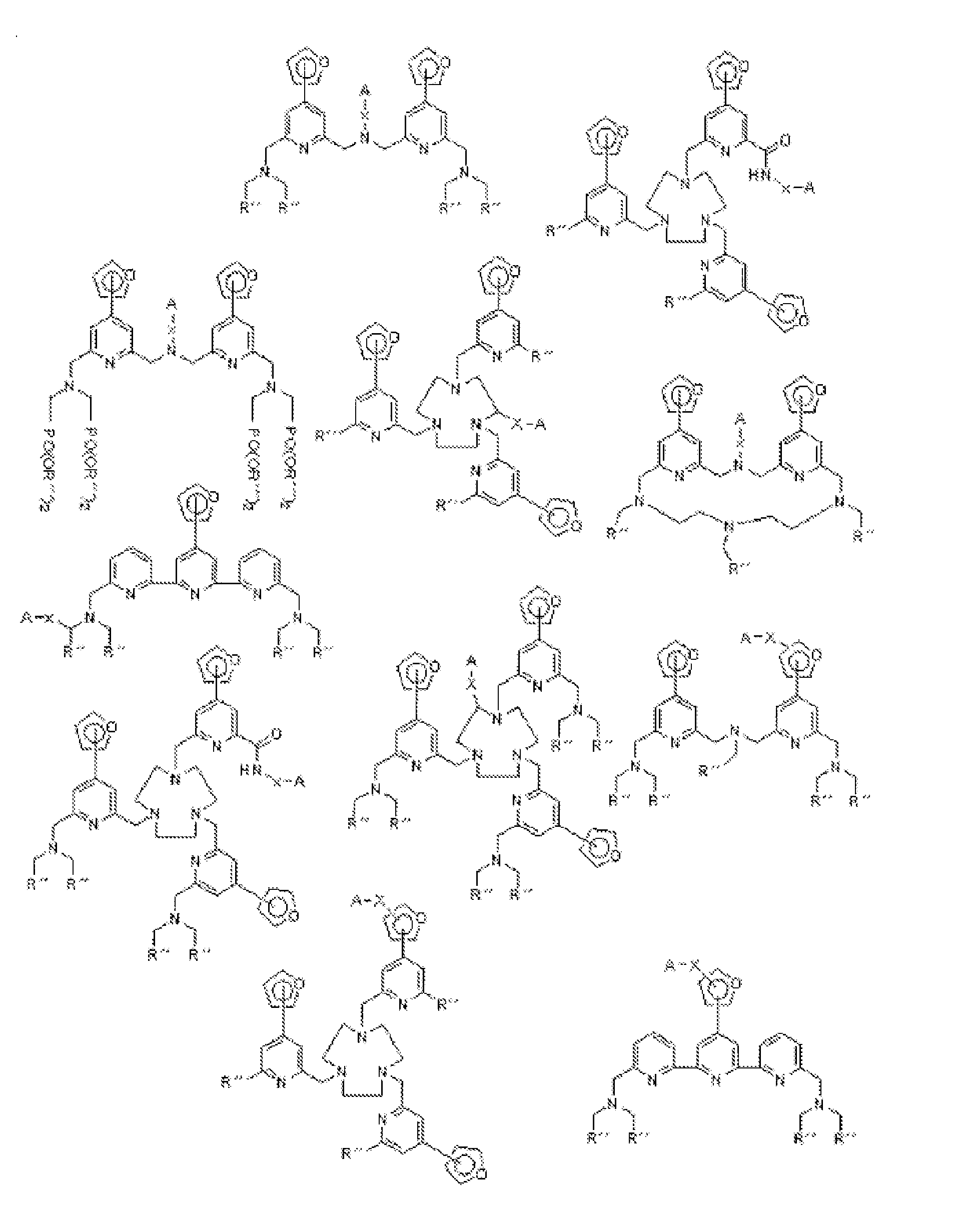 worin x wie in Anspruch 5 definiert ist und die Schutzgruppe R₁ ausgewählt ist aus der Gruppe bestehend aus Fmoc, Boc, oder Bsmoc, und R" ein Alkylester oder ein Allylester und R'" eine Alkylgruppe ist.

7. Ein Chelatbildner umfassend
- eine chromophore Gruppe,
- einen chelatbildenden Teil umfassend mindestens zwei Carbonsäure- oder Phosphonsäureestergruppen, welche entweder direkt oder über eine N-haltige Kohlenwasserstoffkette an die aromatische Einheit der chromophoren Gruppe gebunden sind,
- eine reaktive Gruppe A, gebunden an die chromophore Gruppe oder den chelatbildenden Teil über einen Linker x, wobei
(i) die chromophore Gruppe zwei oder drei Pyridyl-Gruppen umfasst, wobei zumindest eine von diesen furylsubstituiert ist, und besagte Pyridylgruppen entweder direkt aneinander gebunden sind und so eine Terpyridyl-Gruppe bilden, oder über N-haltige Kohlenwasserstoffketten aneinander gebunden sind,
(ii) die reaktive Gruppe A ist
-Y-O-PZ-O-R₄
wobei
eines der Sauerstoffatome optional durch Schwefel ersetzt ist,
Z Chlor oder NR₂R₃ ist,
R₄ eine Schutzgruppe ist,
R₂ und R₃ Alkylgruppen sind,
Y fehlt oder ein Radikal einer Purinbase oder einer Pyrimidinbase oder jeder anderen modifizierten Base geeignet für Verwendung in der Synthese von modifizierten Oligonukleotiden ist, wobei die besagte Base mit dem Sauerstoffatom entweder über
a) eine Kohlenwasserstoffkette, welche mit einer geschützten Hydroxyethylgruppe substituiert ist, oder über
b) einen Furanring oder Pyranring oder jeden modifizierten Furan- oder Pyranring geeignet für Verwendung in der Synthese von modifizierten Oligonukleotiden
verbunden ist,
(iii) der Linker x aus einer bis zehn Einheiten zusammengesetzt ist, wobei jede Einheit ausgewählt ist aus der Gruppe bestehend aus Phenylen, Alkylen mit 1-12 Kohlenstoffatomen, Ethinyldiyl (-C≡C-), Ethylendiyl (-C=C-), Ether (-O-), Thioether (-S-), Amid (-CO-NH-, - CO-NR'-, -NH-CO- und -NR'-CO-), Carbonyl (-CO-), Ester (-COO- und -OOC-), Disulfid (-SS-), Diaza (-N=N-), und tertiärem Amin, worin R' eine Alkylgruppe mit weniger als 5 Kohlenstoffatomen bedeutet.

8. Der Chelatbildner gemäß Anspruch 7, worin Y ein Radikal von irgendeiner der Basen Thymin, Uracil, Adenosin, Guanin oder Cytosin ist, und besagte Base mit dem Sauerstoffatom entweder über
i) eine Kohlenwasserstoffkette, welche mit einer geschützten Hydroxyethylgruppe substituiert ist, oder
ii) einen Furanring mit einer geschützten Hydroxyethylgruppe in seiner 4-Position und optional einer Hydroxyl-, geschützten Hydroxyl- oder modifizierten Hydroxylgruppe in seiner 2-Position
verbunden ist.

9. Der Chelatbildner gemäß Anspruch 8, worin -Y-O-P(NR₂R₃)-O-R₄ ausgewählt ist aus der Gruppe bestehend aus 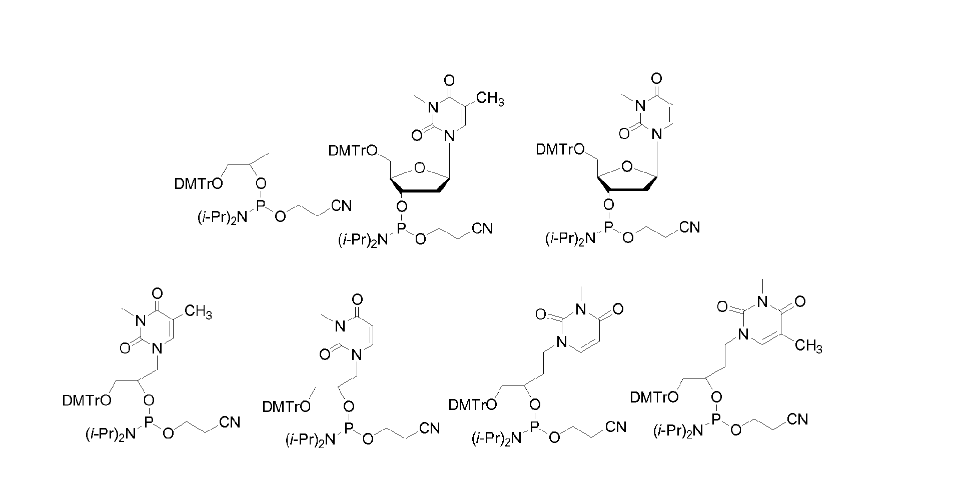 worin "-" die Position des Linkers x und DMTr Dimethoxytrityl ist.

10. Der Chelatbildner gemäß Anspruch 9, ausgewählt aus der Gruppe bestehend aus 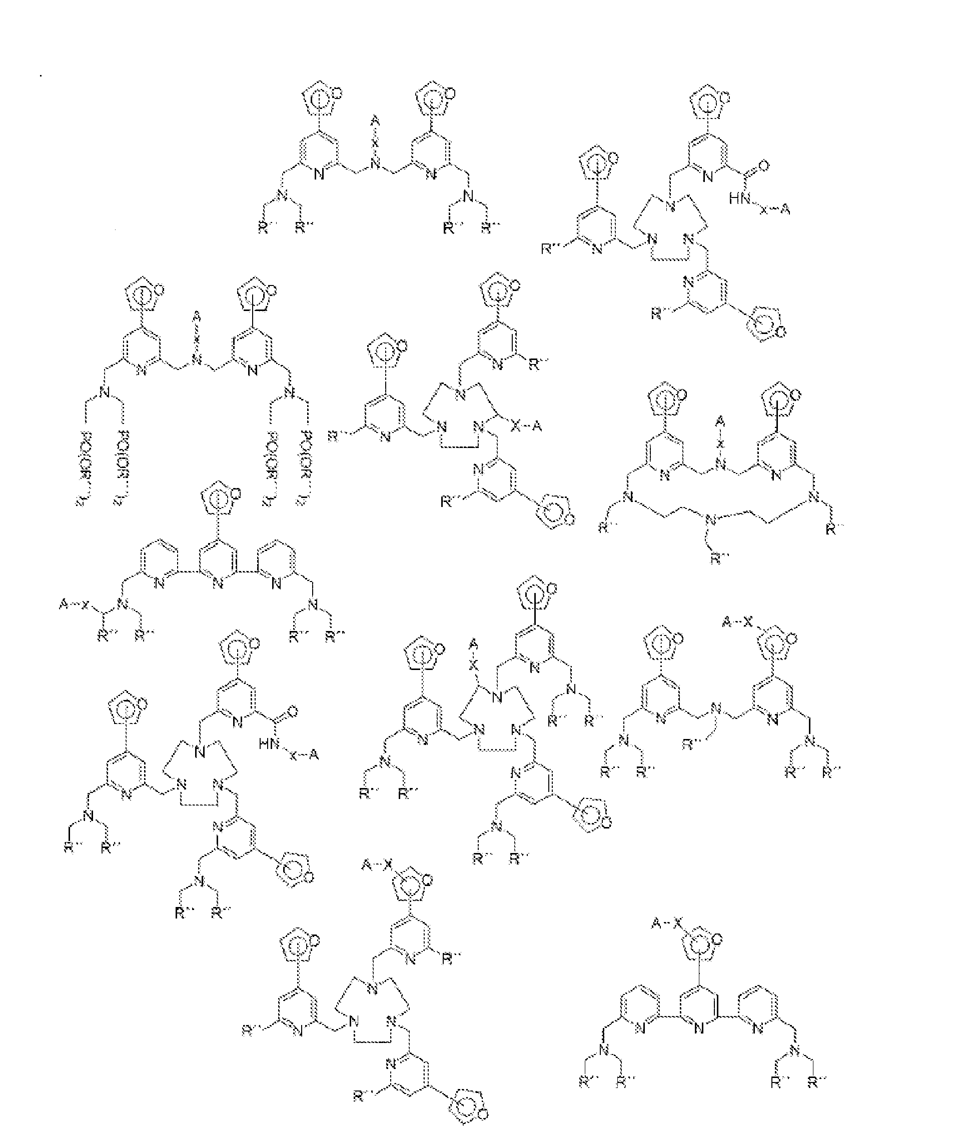 worin R" ein Alkylester oder ein Allylester und R'" eine Alkylgruppe ist und x wie in Anspruch 1 definiert ist und A -Y-O-P(NR₂R₃)-O-R₄ wie in Anspruch 7 definiert ist.

11. Ein Biomolekül konjugiert mit einem Chelat gemäß einem der Ansprüche 1-4.

12. Ein Biomolekül konjugiert mit einem Chelat oder einem Chelatbildner gemäß einem der Ansprüche 1-10, wobei das Biomolekül ausgewählt ist aus der Gruppe bestehend aus einem Oligopeptid, Oligonukleotid, DNA, RNA, modifizierten Oligo- oder Polynukleotid, Protein, Oligosaccharid, Polysaccharid, Phospholipid, PNA, LNA, Antikörper, Hapten, Arzneimittel, Rezeptorbindungsligand und Lectin.

13. Das Biomolekül gemäß Anspruch 12, worin das modifizierte Oligo- oder Polynukleotid ein Phosphoromonothioat, Phosphorodithioat, Phosphoroamidat und/oder ein zucker- oder basenmodifiziertes Oligo- oder Polynukleotid ist.

14. Ein Biomolekül konjugiert mit einem Chelatbildner gemäß einem der Ansprüche 5-10.

15. Ein festes Trägermaterial konjugiert mit einem Chelat gemäß einem der Ansprüche 1-4.

16. Ein festes Trägermaterial konjugiert mit einem Chelat gemäß einem der Ansprüche 1-4, wobei besagtes festes Trägermaterial aus der Gruppe bestehend aus einem Nanopartikel, Mikropartikel, Objektträger oder einer Platte ausgewählt ist.

17. Ein markiertes chelat-gekoppeltes Oligopeptid, erhalten durch Synthese auf einer festen Phase durch Einführung eines Chelatbildners gemäß Anspruch 5 oder 6 in die Oligopeptidstruktur auf einem Oligopeptid-Synthesizer, gefolgt von Entschützung und optionaler Einführung eines Metall-Ions.

18. Ein markiertes chelat-gekoppeltes Oligonukleotid, erhalten durch Synthese auf einer festen Phase durch Einführung eines Chelatbildners gemäß einem der Ansprüche 7-10 in die Oligonukleotidstruktur auf einem Oligonukleotid-Synthesizer, gefolgt von Entschützung und optionaler Einführung eines Metall-Ions.

19. Ein festes Trägermaterial konjugiert mit einem markierten Oligopeptid gemäß Anspruch 17, oder mit einem markierten Oligonukleotid gemäß Anspruch 18, wobei besagtes Oligopeptid oder Oligonukleotid kovalent oder nicht-kovalent auf besagtem festen Trägermaterial immobilisiert ist.

20. Ein festes Trägermaterial konjugiert mit einem markierten Oligopeptid gemäß Anspruch 17, oder mit einem markierten Oligonukleotid gemäß Anspruch 18, wobei besagtes Oligopeptid oder Oligonukleotid kovalent oder nicht-kovalent auf besagtem festen Trägermaterial immobilisiert ist, welches aus der Gruppe bestehend aus einem Nanopartikel, Mikropartikel, Objektträger oder einer Platte ausgewählt ist.

21. Ein festes Trägermaterial konjugiert mit dem Chelatbildner gemäß Anspruch 5, geeignet für Verwendung in der Synthese von einem Oligonukleotid, worin die reaktive Gruppe A
-Y-O-x'-
ist, worin x' ein mit dem festen Tägermaterial verbundener Linker ist, der gleich oder verschieden zum Linker x sein kann,
Y fehlt oder ist ein Radikal einer Purinbase oder einer Pyrimidinbase oder jeder anderen modifizierten Base geeignet für Verwendung in der Synthese von modifizierten Oligonukleotiden, wobei die besagte Base mit dem Sauerstoffatom entweder über
i) eine Kohlenwasserstoffkette, welche mit einer geschützten Hydroxyethylgruppe substituiert ist, oder über
ii) einen Furanring oder Pyranring oder jeden modifizierten Furan- oder Pyranring geeignet für Verwendung in der Synthese von modifizierten Oligonukleotiden
verbunden ist.

## Revendications

1. Chélate comprenant
- un ion lanthanide,
- un fragment chromophore,
- une partie chélatante comprenant au moins deux groupes acide carboxylique ou acide phosphonique, ou des esters ou sels desdits acides, attachée à un motif aromatique du fragment chromophore, soit directement soit par l'intermédiaire d'une chaîne hydrocarbonée contenant N, et
- un groupe réactif A, attaché au fragment chromophore ou à la partie chélatante par l'intermédiaire d'un lieur x, dans lequel
(i) le fragment chromophore comprend deux ou trois groupes pyridyles, dont au moins l'un d'entre eux est à substitution furyle et lesdits groupes pyridyles étant attachés soit directement entre eux pour former un groupe terpyridyle soit entre eux par l'intermédiaire de chaînes hydrocarbonées contenant N,
(ii) le lieur x est formé d'un à dix fragments, chaque fragment étant choisi dans le groupe constitué par un phénylène, un alkylène contenant 1 à 12 atomes de carbone, un éthynyldiyle (-C≡C-), un éthylènediyle (-C=C-), un éther (-0-), un thioéther (-S-), un amide (-CO-NH-, -CO-NR'-, NH-CO et -NR'-CO-), un carbonyle (-CO-), un ester (-COO- et -OOC-), un disulfure (-SS-), un diaza (-N=N-) et une amine tertiaire, où R' représente un groupe alkyle contenant moins de 5 atomes de carbone,
(iii) le groupe réactif A est choisi dans le groupe constitué par un isothiocyanate, un halogénoacétamido, un maléimido, un dichlorotriazinyle, un dichlorotriazinylamino, un pyridyldithio, un thioester, un amino-oxy, un hydrazide, un amino, un groupe polymérisant, et un acide carboxylique ou halogénure d'acide ou un ester actif de ceux-ci.

2. Chélate selon la revendication 1, choisi dans le groupe constitué par 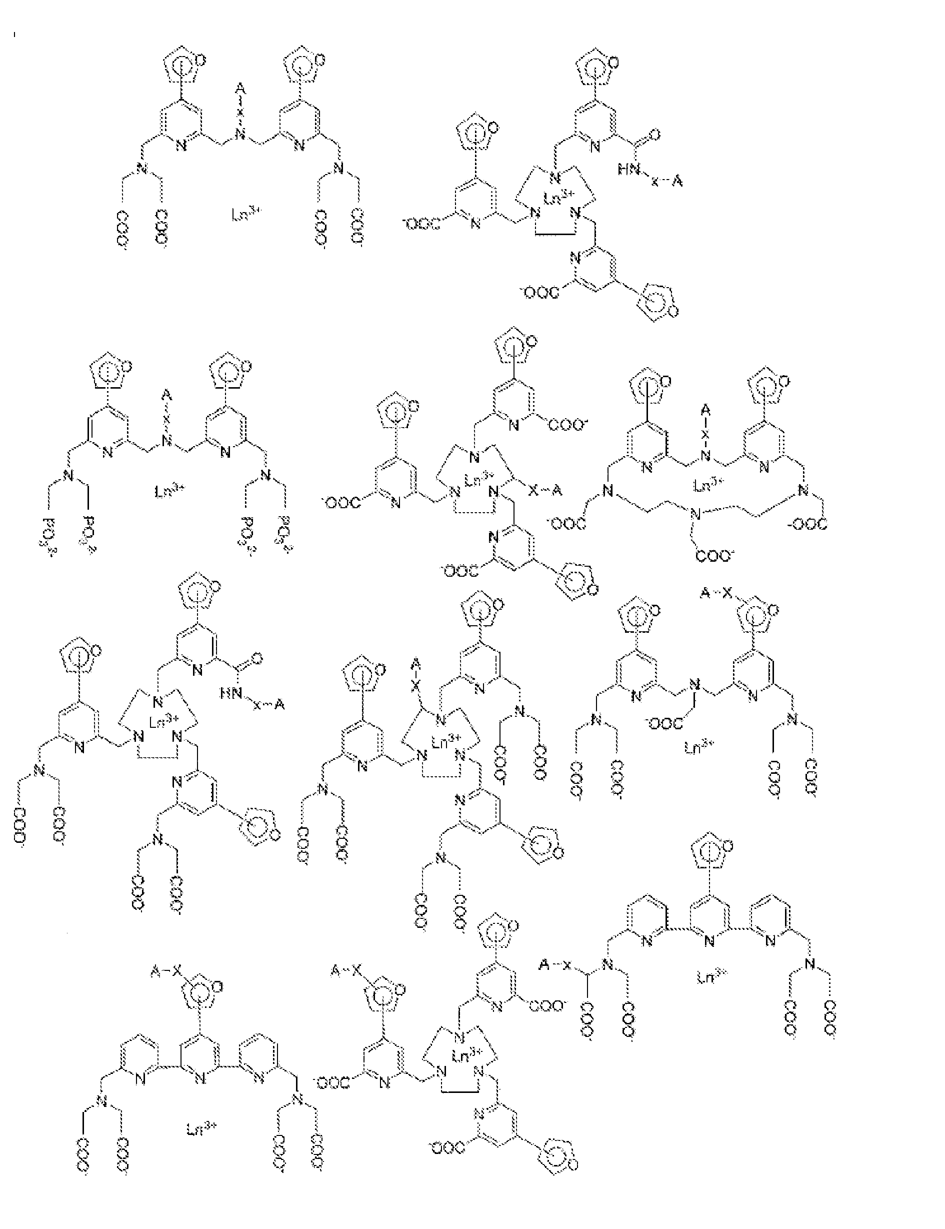 où x et A sont tels que définis dans la revendication 1.

3. Chélate selon la revendication 1, choisi dans le
groupe constitué par 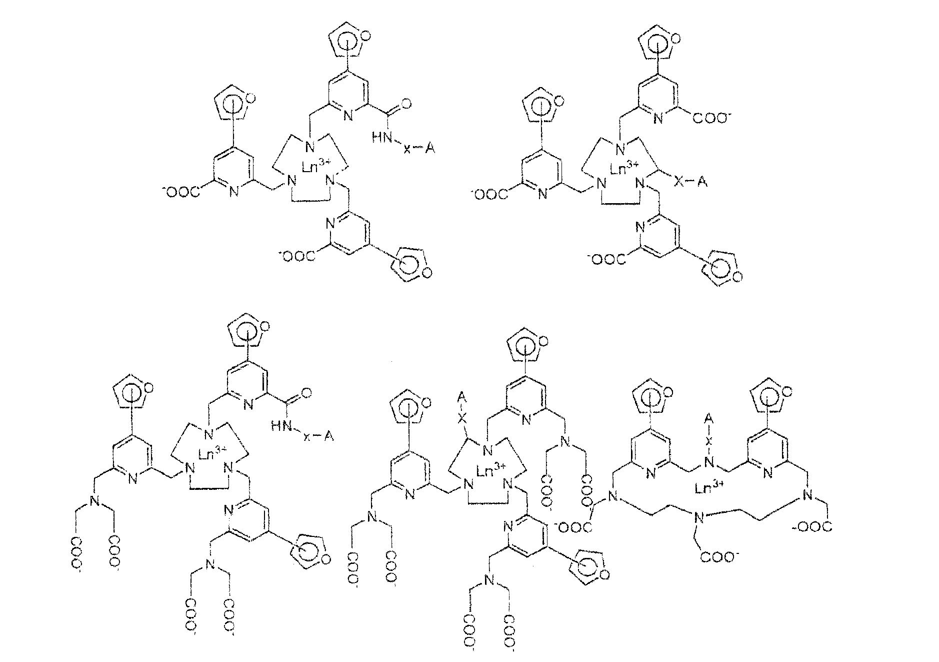 où x et A sont tels que définis dans la revendication 1.

4. Chélate selon les revendications 1 à 3, dans lequel l'ion lanthanide est l'ion europium, samarium, terbium ou dysprosium.

5. Agent chélatant, comprenant
- un fragment chromophore,
- une partie chélatante comprenant au moins deux groupes ester d'acide carboxylique ou d'acide phosphonique, attachée à un motif aromatique du fragment chromophore, soit directement soit par l'intermédiaire d'une chaîne hydrocarbonée contenant N, et
- un groupe réactif A, attaché au fragment chromophore ou à la partie chélatante par l'intermédiaire d'un lieur x, dans lequel
(i) le fragment chromophore comprend deux ou trois groupes pyridyles, dont au moins l'un d'entre eux est à substitution furyle et lesdits groupes pyridyles étant attachés soit directement entre eux pour former un groupe terpyridyle soit entre eux par l'intermédiaire de chaînes hydrocarbonées contenant N,
(ii) le lieur x est formé d'un à dix fragments, chaque fragment étant choisi dans le groupe constitué par un phénylène, un alkylène contenant 1 à 12 atomes de carbone, un éthynyldiyle (-C≡C-), un éthylènediyle (-C=C-), un éther (-0-), un thioéther (-S-), un amide (-CO-NH-, -CO-NR'-, NH-CO et -NR'-CO-), un carbonyle (-CO-), un ester (-COO- et -OOC-), un disulfure (-SS-), un diaza (-N=N-) et une amine tertiaire, où R' représente un groupe alkyle contenant moins de 5 atomes de carbone,
(iii) A est un résidu d'acide aminé -CH(NHR₁)R₅ où R₁ est un groupe protecteur transitoire et R₅ est un acide carboxylique ou son sel, un halogénure d'acide ou un ester.

6. Agent chélatant selon la revendication 5, choisi dans un groupe constitué par 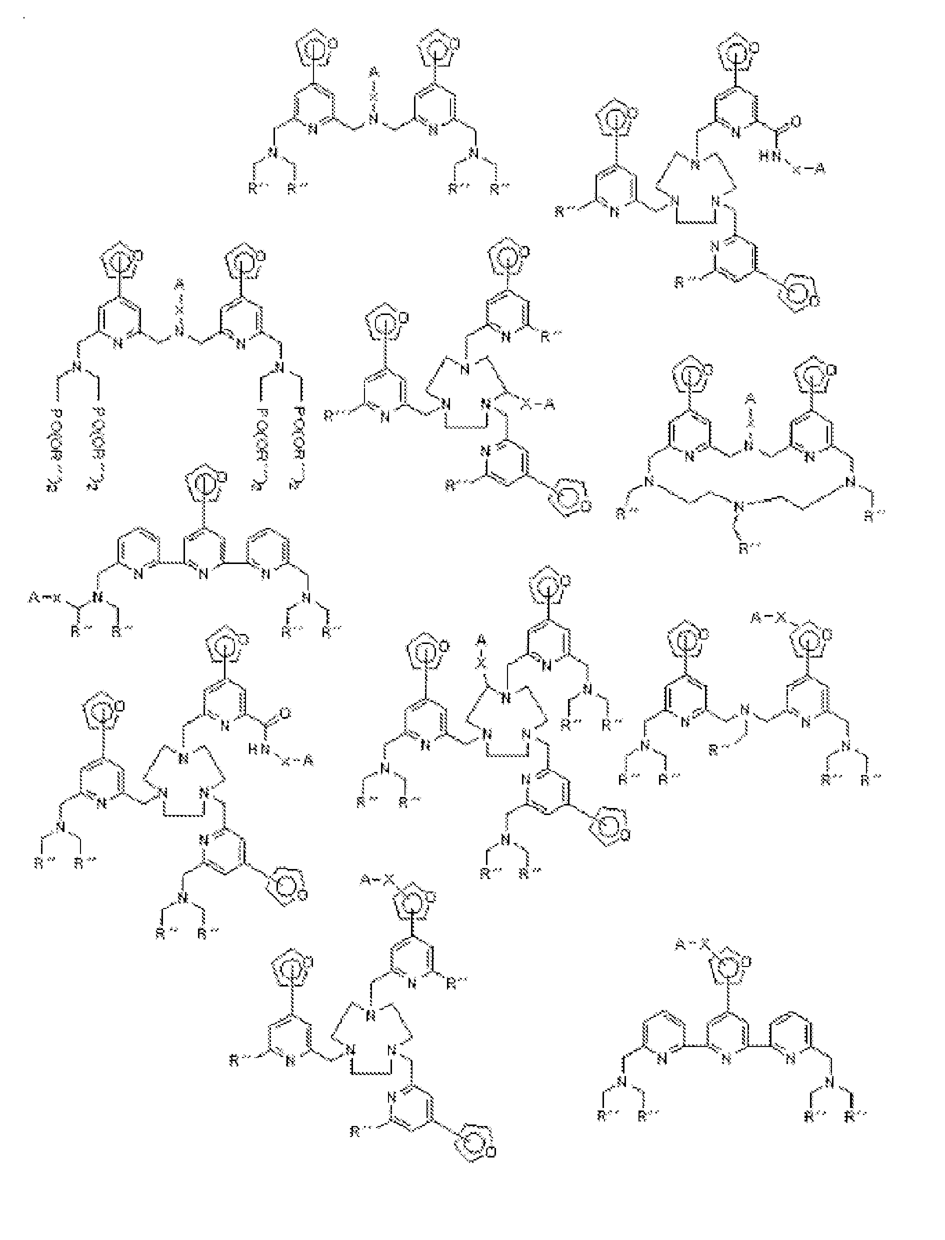 où x est tel que défini dans la revendication 5 et le groupe protecteur R₁ est choisi dans un groupe constitué par Fmoc, Boc ou Bsmoc, et R" est un ester d'alkyle ou un ester d'allyle et R"' est un groupe alkyle.

7. Agent chélatant, comprenant
- un fragment chromophore,
- une partie chélatante comprenant au moins deux groupes ester d'acide carboxylique ou d'acide phosphonique, attachée à un motif aromatique du fragment chromophore, soit directement soit par l'intermédiaire d'une chaîne hydrocarbonée contenant N,
- un groupe réactif A, attaché au fragment chromophore ou à la partie chélatante par l'intermédiaire d'un lieur x, dans lequel
(i) le fragment chromophore comprend deux ou trois groupes pyridyles, dont au moins l'un d'entre eux est à substitution furyle et lesdits groupes pyridyles étant attachés soit directement entre eux pour former un groupe terpyridyle soit entre eux par l'intermédiaire de chaînes hydrocarbonées contenant N,
(ii) le groupe réactif A est
-Y-O-PZ-O-R₄
où
l'un des atomes d'oxygène éventuellement est remplacé par du soufre,
Z est un chloro ou NR₂R₃
R₄ est un groupe protecteur,
R₂ et R₃ sont des groupes alkyles,
Y est absent ou est un radical d'une base purique ou d'une base pyrimidique ou toute autre base modifiée convenant à l'utilisation dans la synthèse d'oligonucléotides modifiés, ladite base étant raccordée à l'atome d'oxygène par l'intermédiaire de
a) soit une chaîne hydrocarbonée, qui est substituée par un groupe hydroxyéthyle protégé,
b) soit par l'intermédiaire d'un cycle furanne ou d'un cycle pyranne ou tout cycle furanne ou pyranne modifié, convenant à l'utilisation dans la synthèse d'oligonucléotides modifiés,
(iii) le lieur x est formé d'un à dix fragments, chaque fragment étant choisi dans le groupe constitué par un phénylène, un alkylène contenant 1 à 12 atomes de carbone, un éthynyldiyle (-C≡C-), un éthylènediyle (-C=C-), un éther (-0-), un thioéther (-S-), un amide (-CO-NH-, -CO-NR'-, NH-CO et -NR'-CO-), un carbonyle (-CO-), un ester (-COO- et -OOC-), un disulfure (-SS-), un diaza (-N=N-) et une amine tertiaire, où R' représente un groupe alkyle contenant moins de 5 atomes de carbone.

8. Agent chélatant selon la revendication 7, dans lequel Y est un radical de l'une quelconque des bases thymine, uracile, adénosine, guanine ou cytosine, et ladite base est raccordée à l'atome d'oxygène par l'intermédiaire
i) d'une chaîne hydrocarbonée, qui est substituée par un groupe hydroxyéthyle protégé, ou
ii) d'un cycle furanne ayant un groupe hydroxyéthyle protégé en sa position 4, et éventuellement un groupe hydroxyle, hydroxyle protégé ou hydroxyle modifié en sa position 2.

9. Agent chélatant selon la revendication 8, dans lequel -Y-O-P (NR₂R₃) -O-R₄ est choisi dans le groupe constitué par 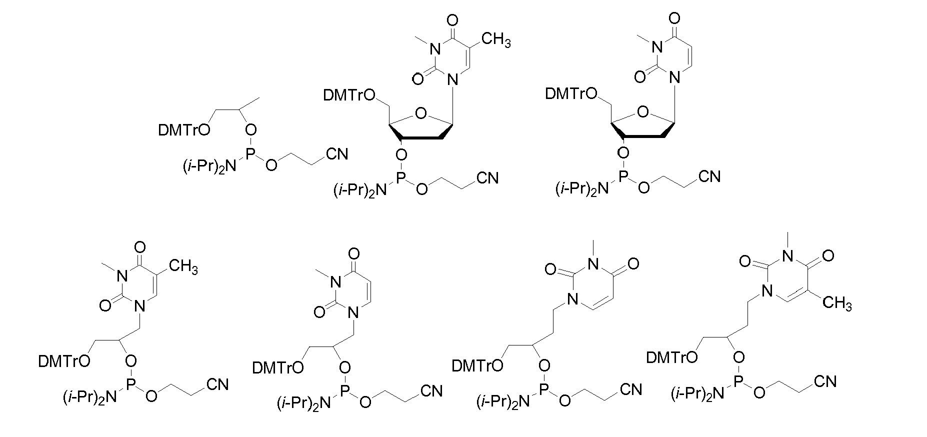 où "-" est la position du lieur x et DMTr est un diméthoxytrityle.

10. Agent chélatant selon la revendication 9, choisi dans le groupe constitué par 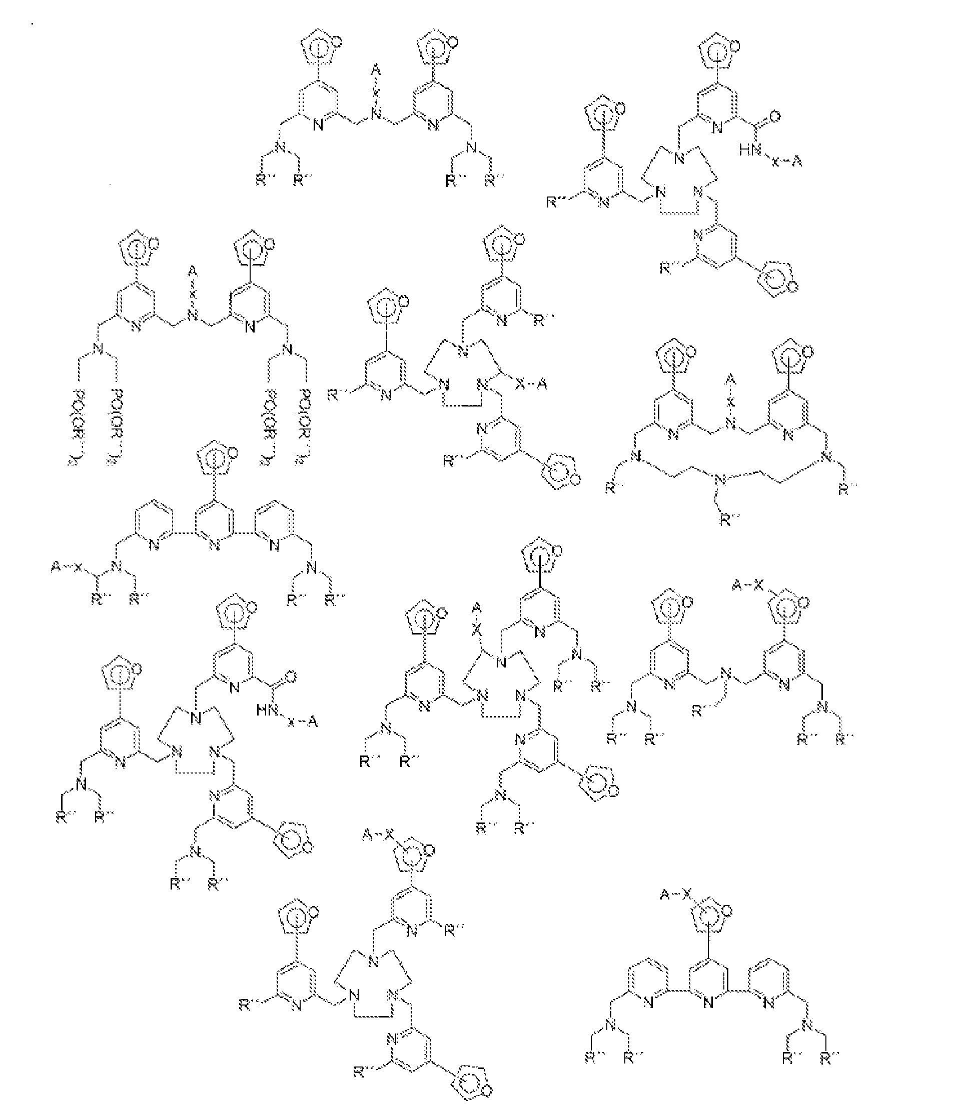 où R" est un ester d'alkyle ou un ester d'allyle et R"' est un groupe alkyle et x est tel que défini dans la revendication 1 et A est -Y-O-P(NR₂R₃)-O-R₄ tel que défini dans la revendication 7.

11. Biomolécule conjuguée avec un chélate selon l'une quelconque des revendications 1 à 4.

12. Biomolécule conjuguée avec un chélate ou un agent chélatant selon l'une quelconque des revendications 1 à 10, dans laquelle la biomolécule est choisie dans le groupe constitué par un oligopeptide, un oligonucléotide, un ADN, un ARN, un oligo- ou polynucléotide modifié, une protéine, un oligosaccharide, un polysaccharide, un phospholipide, un PNA, un LNA, un anticorps, un haptène, une substance médicamenteuse, un ligand de liaison à un récepteur et une lectine.

13. Biomolécule selon la revendication 12, dans laquelle l'oligo- ou polynucléotide modifié est un phosphoromonothioate, un phosphorodithioate, un phosphoroamidate et/ou un oligo- ou polynucléotide modifié par un sucre ou une base.

14. Biomolécule conjuguée avec un agent chélatant selon l'une quelconque des revendications 5 à 10.

15. Support solide conjugué avec un chélate selon l'une quelconque des revendications 1 à 4.

16. Support solide conjugué avec un chélate selon l'une quelconque des revendications 1 à 4, dans lequel ledit support solide est choisi dans le groupe constitué par une nanoparticule, une microparticule, une lame ou une plaque.

17. Oligopeptide marqué couplé à un chélate, obtenu par synthèse sur une phase solide, par l'introduction d'un agent chélatant selon la revendication 5 ou 6 dans la structure oligopeptidique sur un synthétiseur d'oligopeptides, suivie par la déprotection et éventuellement aussi l'introduction d'un ion métallique.

18. Oligonucléotide marqué couplé à un chélate, obtenu par synthèse sur une phase solide, par l'introduction d'un agent chélatant selon l'une quelconque des revendications 7 à 10 dans la structure oligonucléotidique sur un synthétiseur d'oligo-nucléotides, suivie par la déprotection et éventuellement aussi l'introduction d'un ion métallique.

19. Support solide conjugué avec un oligopeptide marqué selon la revendication 17 ou un oligonucléotide marqué selon la revendication 18, dans lesquels ledit oligopeptide ou oligonucléotide est immobilisé de façon covalente ou non covalente sur ledit support solide.

20. Support solide conjugué avec un oligopeptide marqué selon la revendication 17 ou un oligonucléotide marqué selon la revendication 18, dans lesquels ledit oligopeptide ou oligonucléotide est immobilisé de façon covalente ou non covalente sur ledit support solide, qui est choisi dans le groupe constitué par une nanoparticule, une microparticule, une lame ou une plaque.

21. Support solide conjugué avec l'agent chélatant selon la revendication 5, convenant à l'utilisation dans la synthèse d'un oligonucléotide, dans lequel le groupe réactif A est
-Y-O-x'-
où
x' est un lieur raccordé au support solide, et peut être identique au, ou différent du, lieur x
Y est absent ou est un radical d'une purine ou d'une pyrimidine ou de toute autre base modifiée convenant à l'utilisation dans la synthèse d'oligonucléotides modifiés, ladite base étant raccordée à l'atome d'oxygène par l'intermédiaire de
i) soit une chaîne hydrocarbonée, qui est substituée par un groupe hydroxyéthyle protégé,
ii) soit un cycle furanne ou un cycle pyranne ou tout cycle furanne ou pyranne modifié, convenant à l'utilisation dans la synthèse d'oligonucléotides modifiés.
